# EUROPÄISCHE PATENTSCHRIFT

(11) **EP 2 811 028 B1**
(45) Veröffentlichungstag und Bekanntmachung des Hinweises auf die Patenterteilung: **01.02.2017**
(21) Anmeldenummer: 13170248.2
(22) Anmeldetag: 03.06.2013
(51) Int. Cl.: C12P 7/40, C12P 13/08, C12N 15/77

(54) **Verfahren zur Herstellung von L-Valin unter Verwendung rekombinanter Corynebakterien enthaltend das durch Propionat induzierbare ilvBN-Operon**
Process for producing L-valine employing recombinant Corynebacteria comprising the propionate-inducible ilvBN operon
Procédé de production de L-valine en utilisant des Corynebactéries recombinantes comprenant l'opéron ilvBN inductible par le propionate

(43) Veröffentlichungstag der Anmeldung: 10.12.2014
(73) Patentinhaber: Evonik Degussa GmbH, 45128 Essen (DE)
(72) Erfinder: Gerstmeir, Dr.Robert, 33824 Werther (DE); Ramos-Vera, Dr. Hugo, 33611 Bielefeld (DE); Marin, Dr. Kay, 33829 Borgholzhausen (DE)

(56) Entgegenhaltungen:
- EP-A1- 1 096 010
- DE-A1- 10 110 344
- DE-A1-102011 118 019
- PLASSMEIER, J.K. ET AL.: "A propionate-inducible expression system based on the Corynebacterium glutamicum prpD2 promoter and PrpR activator and its application for the redirection of amino acid biosynthesis pathways", JOURNAL OF BIOTECHNOLOGY, Bd. 163, Nr. 2, 20. Januar 2013 (2013-01-20), Seiten 225-232, XP002714643,
- PLASSMEIER J. ET AL.: "Molecular characterization of PrpR, the transcriptional activator of propionate catabolism in Corynebacterium glutamicum", JOURNAL OF BIOTECHNOLOGY, Bd. 159, Nr. 1-2, 31. Mai 2012 (2012-05-31), Seiten 1-11, XP002714644,
- DATABASE EMBL [Online] 15. April 2005 (2005-04-15), CLAES W.A. ET AL:: "Corynebacterium glutamicum prpDBC2 gene cluster, complete sequence", XP002714645, Database accession no. AF434799

### SEQUENCE LISTING

<110> Evonik Industries AG
<120> Optimiertes Verfahren zur Herstellung von Feinchemikalien unter Verwendung eines durch Propionat induzierbaren Promotors
<130> 201100446
<160> 16
<170> PatentIn version 3.5
<210> 1
   <211> 177
   <212> DNA
   <213> Corynebacterium glutamicum
<220>
   <221> repeat_region
   <222> (22)..(49)
   <223> IR1
<220>
   <221> repeat_region
   <222> (77)..(105)
   <223> IR2
<400> 1
   ctccagcgtc cacgaatatg cccccgcgcg ccgggtgggg agcgaaggga acccccaagg 60
   aattggcgtt gaggtggcga ttttgcatgt tttactcaaa attactttgg tggtcacaaa 120
   attacacaac ttttacagtg acctagatcg ctttttaaag aattagcgtg gtgtgca 177
<210> 2
   <211> 177
   <212> DNA
   <213> Corynebacterium glutamicum
<220>
   <221> repeat_region
   <222> (22)..(49)
   <223> IR1
<220>
   <221> repeat_region
   <222> (77)..(105)
   <223> IR2
<400> 2
   ctccagcgtc caagaatatg cccccgcgcg ccgggtgggg agcgaaggga acccccaagg 60
   aattggcgtt gaggtggtga ttttgcatgt tttactcaaa atcactttga tggtcacaaa 120
   attacacaac ttttacagtg acctacattg ctttttaaag aattagtgtg gtgtgca 177
<210> 3
   <211> 177
   <212> DNA
   <213> Corynebacterium glutamicum
<220>
   <221> repeat_region
   <222> (22)..(49)
   <223> IR1
<220>
   <221> repeat_region
   <222> (77)..(105)
   <223> IR2
<400> 3
   ctccagcgtc caagaatatg cccccgcgcg ccgggtgggg agcgaaggga acccccaagg 60
   aattggcgtt gaggtggcga ttttgcatgt tttactcaaa atcactgtga tggtcacaaa 120
   attacacaac ttttacagtg acctagatcg ctttttaaag aattagcgtg gtgtgca 177
<210> 4
   <211> 902
   <212> DNA
   <213> Corynebacterium glutamicum
<220>
   <221> promoter
   <222> (1)..(177)
   <223> PprpD2
<220>
   <221> RBS
   <222> (178)..(206)
   <223> gap RBS
<220>
   <221> misc_feature
   <222> (207)..(209)
   <223> atg-Startcodon
<220>
   <221> gene
   <222> (207)..(902)
   <223> ilvB-Kodierregion 5'-Region
<400> 4
   ctccagcgtc cacgaatatg cccccgcgcg ccgggtgggg agcgaaggga acccccaagg 60
   aattggcgtt gaggtggcga ttttgcatgt tttactcaaa attactttgg tggtcacaaa 120
   attacacaac ttttacagtg acctagatcg ctttttaaag aattagcgtg gtgtgcatgc 180
   atatttatcg cgagaggaga cacaacatga atgtggcagc ttctcaacag cccactcccg 240
   ccacggttgc aagccgtggt cgatccgccg cccctgagcg gatgacaggt gcacaggcaa 300
   ttgttcgatc gctcgaggag cttaacgccg acatcgtgtt cggtattcct ggtggtgcgg 360
   tgctaccggt gtatgacccg ctctattcct ccacaaaggt gcgccacgtc ctggtgcgcc 420
   acgagcaggg cgcaggccac gcagcaaccg gctacgcgca ggttactgga cgcgttggcg 480
   tctgcattgc aacctctggc ccaggcgcaa ccaacttggt taccccaatc gctgatgcaa 540
   acttggactc cgttcccatg gttgccatca ccggccaggt cggaagtggc ctgctgggta 600
   ccgatgcttt ccaggaagcc gatatccgcg gcatcaccat gccagtgacc aagcacaact 660
   tcatggtcac cgaccccaac gacattccac aggcattggc tgaggcattc cacctcgcga 720
   ttactggtcg ccctggccct gttctggtgg atattcctaa ggatgtccaa aacgctgaat 780
   tggatttcgt ctggccacca aagatcgacc tgccaggcta ccgcccagtt tctactccgc 840
   atgctcgaca gattgagcag gctgtcaaac tgatcggtga agccaaaaag ccagtccttt 900
   ac 902
<210> 5
   <211> 1390
   <212> DNA
   <213> Corynebacterium glutamicum
<220>
   <221> misc_feature
   <222> (494)..(521)
   <223> IR1
<220>
   <221> misc_feature
   <222> (549)..(577)
   <223> IR2
<220>
   <221> RBS
   <222> (665)..(678)
   <223> RBS gap
<220>
   <221> gene
   <222> (679)..(1380)
   <223> ilvBN 5'-region
<400> 5
   cgaattcgtg caatcgtgcg aattttggtc gcgcgcatgg tgatctcaat gacggtgcct 60
   tcgacaacga tgccgttgcc ctcaaaacgc acccagtcac ccacgccgaa ttgcttttcc 120
   gtcaggatga aaaatccggc caagaagtcc gcaacaatcg actgcgcacc aaggccaatg 180
   gcagctgacg caatggttgc cggaatcgca gcgcccgcga gagagaaacc aaaagcctgc 240
   atcgcggaga cggcaagcat gaaaaacgcc acaatttgcg cgatataaac gccaacgcca 300
   gcgaacgcga gctggttctt agtggtgtcc gcatcggctg cagactccac tcgccgcttg 360
   ataatacgca tggccagtcg gccgatacgt ggaatcaaaa acgccaagac caggataatt 420
   gctacatcaa aaccggtatc gacaatccaa ttccacaatg aatagagcaa atctccagcg 480
   tccacgaata tgcccccgcg cgccgggtgg ggagcgaagg gaacccccaa ggaattggcg 540
   ttgaggtggc gattttgcat gttttactca aaattacttt ggtggtcaca aaattacaca 600
   acttttacag tgacctagat cgctttttaa agaattagcg tggtgtgcat gcatatttat 660
   cgcgagagga gacacaacat gaatgtggca gcttctcaac agcccactcc cgccacggtt 720
   gcaagccgtg gtcgatccgc cgcccctgag cggatgacag gtgcacaggc aattgttcga 780
   tcgctcgagg agcttaacgc cgacatcgtg ttcggtattc ctggtggtgc ggtgctaccg 840
   gtgtatgacc cgctctattc ctccacaaag gtgcgccacg tcctggtgcg ccacgagcag 900
   ggcgcaggcc acgcagcaac cggctacgcg caggttactg gacgcgttgg cgtctgcatt 960
   gcaacctctg gcccaggcgc aaccaacttg gttaccccaa tcgctgatgc aaacttggac 1020
   tccgttccca tggttgccat caccggccag gtcggaagtg gcctgctggg taccgatgct 1080
   ttccaggaag ccgatatccg cggcatcacc atgccagtga ccaagcacaa cttcatggtc 1140
   accgacccca acgacattcc acaggcattg gctgaggcat tccacctcgc gattactggt 1200
   cgccctggcc ctgttctggt ggatattcct aaggatgtcc aaaacgctga attggatttc 1260
   gtctggccac caaagatcga cctgccaggc taccgcccag tttctactcc gcatgctcga 1320
   cagattgagc aggctgtcaa actgatcggt gaagccaaaa agccagtcct ttacattggc 1380
   ggcaagcttg 1390
<210> 6
   <211> 20
   <212> DNA
   <213> Corynebacterium glutamicum
<220>
   <221> misc_feature
   <222> (1)..(20)
   <223> Testprimer 1
<400> 6
   aaagcctgca tcgcggagac 20
<210> 7
   <211> 20
   <212> DNA
   <213> Corynebacterium glutamicum
<220>
   <221> misc_feature
   <222> (1)..(20)
   <223> Testprimer 2
<400> 7
   tggtgatgcc gcggatatcg 20
<210> 8
   <211> 3411
   <212> DNA
   <213> Corynebacterium glutamicum
<220>
   <221> CDS
   <222> (500)..(2380)
   <223> Kodierregion ilvB
<220>
   <221> CDS
   <222> (2394)..(2912)
   <223> Kodierregion ilvN
<400> 8
   gtatcgacaa tccaattcca caatgaatag agcaaatatt gaatgggtac gcctaaaatc    60
   atgagccaag attagcgctg aaaagtagcg ggagcctgcc tgaactttgt gagaatcctg   120
   attccttaac cgaagtgggg gagttttggg ggtgggaatt ttcgtgcgtt gtggaattgg   180
   aaactcgatg tgtgtagcat gacacaccat gaccattatt cgacttgtag tagtaaccgc   240
   gcggcgcctg ccgtaacggc cttccaagtc gtctcgtcaa gcgccctcga caacactcac   300
   cacagtgttg gaacgagggc tttcttgttg gttatgaccc aagtagccaa ctttgcaaca   360
   gacatctgtc gcactgcgtg cacacgcatc cgcgtcggaa caattttaaa tgagggcttt   420
   gtctttaggc tgagttgaaa tcggcttggc ttggacgggt cctgtgaaaa tccttattta   480
   gtaaaggagc cagaaagtc gtg aat gtg gca gct tct caa cag ccc act ccc    532
                        Val Asn Val Ala Ala Ser Gln Gln Pro Thr Pro
                        1               5                   10
   gcc acg gtt gca agc cgt ggt cga tcc gcc gcc cct gag cgg atg aca     580
   Ala Thr Val Ala Ser Arg Gly Arg Ser Ala Ala Pro Glu Arg Met Thr
               15                  20                  25
   ggt gca aag gca att gtt cga tcg ctc gag gag ctt aac gcc gac atc     628
   Gly Ala Lys Ala Ile Val Arg Ser Leu Glu Glu Leu Asn Ala Asp Ile
           30                  35                  40
   gtg ttc ggt att cct ggt ggt gcg gtg cta ccg gtg tat gac ccg ctc     676
   Val Phe Gly Ile Pro Gly Gly Ala Val Leu Pro Val Tyr Asp Pro Leu
       45                  50                  55
   tat tcc tcc aca aag gtg cgc cac gtc ttg gtg cgc cac gag cag ggc     724
   Tyr Ser Ser Thr Lys Val Arg His Val Leu Val Arg His Glu Gln Gly
   60                  65                  70                  75
   gca ggc cac gca gca acc ggc tac gcg cag gtt act gga cgc gtt ggc     772
   Ala Gly His Ala Ala Thr Gly Tyr Ala Gln Val Thr Gly Arg Val Gly
                   80                  85                  90
   gtc tgc att gca acc tct ggc cca gga gca acc aac ttg gtt acc cca     820
   Val Cys Ile Ala Thr Ser Gly Pro Gly Ala Thr Asn Leu Val Thr Pro
               95                  100                 105
   atc gct gat gca aac ttg gac tcc gtt ccc atg gtt gcc atc acc ggc     868
   Ile Ala Asp Ala Asn Leu Asp Ser Val Pro Met Val Ala Ile Thr Gly
           110                 115                 120
   cag gtc gga agt ggc ctg ctg ggt acc gac gct ttc cag gaa gcc gat        916
   Gln Val Gly Ser Gly Leu Leu Gly Thr Asp Ala Phe Gln Glu Ala Asp
       125                 130                 135
   atc cgc ggc atc acc atg cca gtg acc aag cac aac ttc atg gtc acc        964
   Ile Arg Gly Ile Thr Met Pro Val Thr Lys His Asn Phe Met Val Thr
   140                 145                 150                 155
   aac cct aac gac att cca cag gca ttg gct gag gca ttc cac ctc gcg       1012
   Asn Pro Asn Asp Ile Pro Gln Ala Leu Ala Glu Ala Phe His Leu Ala
                   160                 165                 170
   att act ggt cgc cct ggc cct gtt ctg gtg gat att cct aag gat gtc       1060
   Ile Thr Gly Arg Pro Gly Pro Val Leu Val Asp Ile Pro Lys Asp Val
               175                 180                 185
   cag aac gct gaa ttg gat ttc gtc tgg cca cca aag atc gac ctg cca       1108
   Gln Asn Ala Glu Leu Asp Phe Val Trp Pro Pro Lys Ile Asp Leu Pro
           190                 195                 200
   ggc tac cgc cca gtt tca aca cca cat gct cgc cag atc gag cag gca       1156
   Gly Tyr Arg Pro Val Ser Thr Pro His Ala Arg Gln Ile Glu Gln Ala
       205                 210                 215
   gtc aag ctg atc ggt gag gcc aag aag ccc gtc ctt tac gtt ggt ggt       1204
   Val Lys Leu Ile Gly Glu Ala Lys Lys Pro Val Leu Tyr Val Gly Gly
   220                 225                 230                 235
   ggc gta atc aag gct gac gca cac gaa gag ctt cgt gcg ttc gct gag       1252
   Gly Val Ile Lys Ala Asp Ala His Glu Glu Leu Arg Ala Phe Ala Glu
                   240                 245                 250
   tac acc ggc atc cca gtt gtc acc acc ttg atg gct ttg ggt act ttc       1300
   Tyr Thr Gly Ile Pro Val Val Thr Thr Leu Met Ala Leu Gly Thr Phe
               255                 260                 265
   cca gag tct cac gag ctg cac atg ggt atg cca ggc atg cat ggc act       1348
   Pro Glu Ser His Glu Leu His Met Gly Met Pro Gly Met His Gly Thr
           270                 275                 280
   gtg tcc gct gtt ggt gca ctg cag cgc agc gac ctg ctg att gct atc       1396
   Val Ser Ala Val Gly Ala Leu Gln Arg Ser Asp Leu Leu Ile Ala Ile
       285                 290                 295
   ggc tcc cgc ttt gat gac cgc gtc acc ggt gac gtt gac acc ttc gcg       1444
   Gly Ser Arg Phe Asp Asp Arg Val Thr Gly Asp Val Asp Thr Phe Ala
   300                 305                 310                 315
   cct gac gcc aag atc att cac gcc gac att gat cct gcc gaa atc ggc       1492
   Pro Asp Ala Lys Ile Ile His Ala Asp Ile Asp Pro Ala Glu Ile Gly
                   320                 325                 330
   aag atc aag cag gtt gag gtt cca atc gtg ggc gat gcc cgc gaa gtt       1540
   Lys Ile Lys Gln Val Glu Val Pro Ile Val Gly Asp Ala Arg Glu Val
               335                 340                 345
   ctt gct cgt ctg ctg gaa acc acc aag gca agc aag gca gag acc gag       1588
   Leu Ala Arg Leu Leu Glu Thr Thr Lys Ala Ser Lys Ala Glu Thr Glu
           350                 355                 360
   gac atc tcc gag tgg gtt gac tac ctc aag ggc ctc aag gca cgt ttc       1636
   Asp Ile Ser Glu Trp Val Asp Tyr Leu Lys Gly Leu Lys Ala Arg Phe
       365                 370                 375
   ccg cgt ggc tac gac gag cag cca ggc gat ctg ctg gca cca cag ttt        1684
   Pro Arg Gly Tyr Asp Glu Gln Pro Gly Asp Leu Leu Ala Pro Gln Phe
   380                 385                 390                 395
   gtc att gaa acc ctg tcc aag gaa gtt ggc ccc gac gca att tac tgc        1732
   Val Ile Glu Thr Leu Ser Lys Glu Val Gly Pro Asp Ala Ile Tyr Cys
                   400                 405                 410
   gcc ggc gtt ggc cag cac caa atg tgg gca gct cag ttc gtt gac ttt        1780
   Ala Gly Val Gly Gln His Gln Met Trp Ala Ala Gln Phe Val Asp Phe
               415                 420                 425
   gaa aag cca cgc acc tgg ctc aac tcc ggt gga ctg ggc acc atg ggc        1828
   Glu Lys Pro Arg Thr Trp Leu Asn Ser Gly Gly Leu Gly Thr Met Gly
           430                 435                 440
   tac gca gtt cct gcg gcc ctt gga gca aag gct ggc gca cct gac aag        1876
   Tyr Ala Val Pro Ala Ala Leu Gly Ala Lys Ala Gly Ala Pro Asp Lys
       445                 450                 455
   gaa gtc tgg gct atc gac ggc gac ggc tgt ttc cag atg acc aac cag        1924
   Glu Val Trp Ala Ile Asp Gly Asp Gly Cys Phe Gln Met Thr Asn Gln
   460                 465                 470                 475
   gaa ctc acc acc gcc gca gtt gaa ggt ttc ccc att aag atc gca cta        1972
   Glu Leu Thr Thr Ala Ala Val Glu Gly Phe Pro Ile Lys Ile Ala Leu
                   480                 485                 490
   atc aac aac gga aac ctg ggc atg gtt cgc caa tgg cag acc cta ttc        2020
   Ile Asn Asn Gly Asn Leu Gly Met Val Arg Gln Trp Gln Thr Leu Phe
               495                 500                 505
   tat gaa gga cgg tac tca aat act aaa ctt cgt aac cag ggc gag tac        2068
   Tyr Glu Gly Arg Tyr Ser Asn Thr Lys Leu Arg Asn Gln Gly Glu Tyr
           510                 515                 520
   atg ccc gac ttt gtt acc ctt tct gag gga ctt ggc tgt gtt gcc atc        2116
   Met Pro Asp Phe Val Thr Leu Ser Glu Gly Leu Gly Cys Val Ala Ile
       525                 530                 535
   cgc gtc acc aaa gcg gag gaa gta ctg cca gcc atc caa aag gct cga        2164
   Arg Val Thr Lys Ala Glu Glu Val Leu Pro Ala Ile Gln Lys Ala Arg
   540                 545                 550                 555
   gag atc aac gac cgc cca gta gtc atc gac ttc atc gtc ggt gaa gac        2212
   Glu Ile Asn Asp Arg Pro Val Val Ile Asp Phe Ile Val Gly Glu Asp
                   560                 565                 570
   gca cag gta tgg cca atg gtg tct gct gga tca tcc aac tcc gat atc        2260
   Ala Gln Val Trp Pro Met Val Ser Ala Gly Ser Ser Asn Ser Asp Ile
               575                 580                 585
   cag tac gca ctc gga ttg cgc cca ttc ttt gat ggt gat gaa tct gca        2308
   Gln Tyr Ala Leu Gly Leu Arg Pro Phe Phe Asp Gly Asp Glu Ser Ala
           590                 595                 600
   gca gaa gat cct gcc gac att cac gaa gcc gtc agc gac att gat gcc        2356
   Ala Glu Asp Pro Ala Asp Ile His Glu Ala Val Ser Asp Ile Asp Ala
       605                 610                 615
   gcc gtt gaa tcg acc gag gca taa ggagagaccc aag atg gct aat tct         2405
   Ala Val Glu Ser Thr Glu Ala                     Met Ala Asn Ser
   620                 625                                     630
   gac gtc acc cgc cac atc ctg tcc gta ctc gtt cag gac gta gac gga       2453
   Asp Val Thr Arg His Ile Leu Ser Val Leu Val Gln Asp Val Asp Gly
                   635                 640                 645
   atc att tcc cgc gta tca ggt atg ttc acc cga cgc gca ttc aac ctc       2501
   Ile Ile Ser Arg Val Ser Gly Met Phe Thr Arg Arg Ala Phe Asn Leu
               650                 655                 660
   gtg tcc ctc gtg tct gca aag acc gaa aca cac ggc atc aac cgc atc       2549
   Val Ser Leu Val Ser Ala Lys Thr Glu Thr His Gly Ile Asn Arg Ile
           665                 670                 675
   acg gtt gtt gtc gac gcc gac gag ctc aac att gag cag atc acc aag       2597
   Thr Val Val Val Asp Ala Asp Glu Leu Asn Ile Glu Gln Ile Thr Lys
       680                 685                 690
   cag ctc aac aag ctg atc ccc gtg ctc aaa gtc gtg cga ctt gat gaa       2645
   Gln Leu Asn Lys Leu Ile Pro Val Leu Lys Val Val Arg Leu Asp Glu
   695                 700                 705                 710
   gag acc act atc gcc cgc gca atc atg ctg gtt aag gtc tct gcg gac       2693
   Glu Thr Thr Ile Ala Arg Ala Ile Met Leu Val Lys Val Ser Ala Asp
                   715                 720                 725
   agc acc aac cgt ccg cag atc gtc gac gcc gcg aac atc ttc cgc gcc       2741
   Ser Thr Asn Arg Pro Gln Ile Val Asp Ala Ala Asn Ile Phe Arg Ala
               730                 735                 740
   cga gtc gtc gac gtg gct cca gac tct gtg gtt att gaa tcc aca ggc       2789
   Arg Val Val Asp Val Ala Pro Asp Ser Val Val Ile Glu Ser Thr Gly
           745                 750                 755
   acc cca ggc aag ctc cgc gca ctg ctt gac gtg atg gaa cca ttc gga       2837
   Thr Pro Gly Lys Leu Arg Ala Leu Leu Asp Val Met Glu Pro Phe Gly
       760                 765                 770
   atc cgc gaa ctg atc caa tcc gga cag att gca ctc aac cgc ggt ccg       2885
   Ile Arg Glu Leu Ile Gln Ser Gly Gln Ile Ala Leu Asn Arg Gly Pro
   775                 780                 785                 790
   aag acc atg gct ccg gcc aag atc taa acagcaatta atctgattgc             2932
   Lys Thr Met Ala Pro Ala Lys Ile
                   795
   acctgctgca taaatgtgac tagtcaaaca ccgtctaatt acatgtgtgt ggtagaacaa     2992
   taatgtagtt gtctgcccaa gcgagttaaa ctcccacgat ttacagtggg gggcagacat     3052
   cttttcacca aaatttttac gaaaggcgag attttctccc atggctattg aactgcttta     3112
   tgatgctgac gctgacctct ccttgatcca gggccgtaag gttgccatcg ttggctacgg     3172
   ctcccagggc cacgcacact cccagaacct ccgcgattct ggcgttgagg ttgtcattgg     3232
   tctgcgcgag ggctccaagt ccgcagagaa ggcaaaggaa gcaggcttcg aggtcaagac     3292
   caccgctgag gctgcagctt gggctgacgt catcatgctc ctggctccag acacctccca     3352
   ggcagaaatc ttcaccaacg acatcgagcc aaacctgaac gcaggcgacg cactgctgt      3411
<210> 9
   <211> 626
   <212> PRT
   <213> Corynebacterium glutamicum
<400> 9
   Val Asn Val Ala Ala Ser Gln Gln Pro Thr Pro Ala Thr Val Ala Ser
   1               5                   10                  15
   Arg Gly Arg Ser Ala Ala Pro Glu Arg Met Thr Gly Ala Lys Ala Ile
               20                  25                  30
   Val Arg Ser Leu Glu Glu Leu Asn Ala Asp Ile Val Phe Gly Ile Pro
           35                  40                  45
   Gly Gly Ala Val Leu Pro Val Tyr Asp Pro Leu Tyr Ser Ser Thr Lys
       50                  55                  60
   Val Arg His Val Leu Val Arg His Glu Gln Gly Ala Gly His Ala Ala
   65                  70                  75                  80
   Thr Gly Tyr Ala Gln Val Thr Gly Arg Val Gly Val Cys Ile Ala Thr
                   85                  90                  95
   Ser Gly Pro Gly Ala Thr Asn Leu Val Thr Pro Ile Ala Asp Ala Asn
               100                 105                 110
   Leu Asp Ser Val Pro Met Val Ala Ile Thr Gly Gln Val Gly Ser Gly
           115                 120                 125
   Leu Leu Gly Thr Asp Ala Phe Gln Glu Ala Asp Ile Arg Gly Ile Thr
       130                 135                 140
   Met Pro Val Thr Lys His Asn Phe Met Val Thr Asn Pro Asn Asp Ile
   145                 150                 155                 160
   Pro Gln Ala Leu Ala Glu Ala Phe His Leu Ala Ile Thr Gly Arg Pro
                   165                 170                 175
   Gly Pro Val Leu Val Asp Ile Pro Lys Asp Val Gln Asn Ala Glu Leu
               180                 185                 190
   Asp Phe Val Trp Pro Pro Lys Ile Asp Leu Pro Gly Tyr Arg Pro Val
           195                 200                 205
   Ser Thr Pro His Ala Arg Gln Ile Glu Gln Ala Val Lys Leu Ile Gly
       210                 215                 220
   Glu Ala Lys Lys Pro Val Leu Tyr Val Gly Gly Gly Val Ile Lys Ala
   225 230 235 240
   Asp Ala His Glu Glu Leu Arg Ala Phe Ala Glu Tyr Thr Gly Ile Pro
   245 250 255
   Val Val Thr Thr Leu Met Ala Leu Gly Thr Phe Pro Glu Ser His Glu
   260 265 270
   Leu His Met Gly Met Pro Gly Met His Gly Thr Val Ser Ala Val Gly
   275 280 285
   Ala Leu Gln Arg Ser Asp Leu Leu Ile Ala Ile Gly Ser Arg Phe Asp
   290 295 300
   Asp Arg Val Thr Gly Asp Val Asp Thr Phe Ala Pro Asp Ala Lys Ile
   305 310 315 320
   Ile His Ala Asp Ile Asp Pro Ala Glu Ile Gly Lys Ile Lys Gln Val
   325 330 335
   Glu Val Pro Ile Val Gly Asp Ala Arg Glu Val Leu Ala Arg Leu Leu
   340 345 350
   Glu Thr Thr Lys Ala Ser Lys Ala Glu Thr Glu Asp Ile Ser Glu Trp
   355 360 365
   Val Asp Tyr Leu Lys Gly Leu Lys Ala Arg Phe Pro Arg Gly Tyr Asp
   370 375 380
   Glu Gln Pro Gly Asp Leu Leu Ala Pro Gln Phe Val Ile Glu Thr Leu
   385 390 395 400
   Ser Lys Glu Val Gly Pro Asp Ala Ile Tyr Cys Ala Gly Val Gly Gln
   405 410 415
   His Gln Met Trp Ala Ala Gln Phe Val Asp Phe Glu Lys Pro Arg Thr
   420 425 430
   Trp Leu Asn Ser Gly Gly Leu Gly Thr Met Gly Tyr Ala Val Pro Ala
   435 440 445
   Ala Leu Gly Ala Lys Ala Gly Ala Pro Asp Lys Glu Val Trp Ala Ile
   450 455 460
   Asp Gly Asp Gly Cys Phe Gln Met Thr Asn Gln Glu Leu Thr Thr Ala
   465 470 475 480
   Ala Val Glu Gly Phe Pro Ile Lys Ile Ala Leu Ile Asn Asn Gly Asn
   485 490 495
   Leu Gly Met Val Arg Gln Trp Gln Thr Leu Phe Tyr Glu Gly Arg Tyr
   500 505 510
   Ser Asn Thr Lys Leu Arg Asn Gln Gly Glu Tyr Met Pro Asp Phe Val
   515 520 525
   Thr Leu Ser Glu Gly Leu Gly Cys Val Ala Ile Arg Val Thr Lys Ala
   530 535 540
   Glu Glu Val Leu Pro Ala Ile Gln Lys Ala Arg Glu Ile Asn Asp Arg
   545 550 555 560
   Pro Val Val Ile Asp Phe Ile Val Gly Glu Asp Ala Gln Val Trp Pro
   565 570 575
   Met Val Ser Ala Gly Ser Ser Asn Ser Asp Ile Gln Tyr Ala Leu Gly
   580 585 590
   Leu Arg Pro Phe Phe Asp Gly Asp Glu Ser Ala Ala Glu Asp Pro Ala
   595 600 605
   Asp Ile His Glu Ala Val Ser Asp Ile Asp Ala Ala Val Glu Ser Thr
   610 615 620
   Glu Ala
   625
<210> 10
   <211> 172
   <212> PRT
   <213> Corynebacterium glutamicum
<400> 10
   Met Ala Asn Ser Asp Val Thr Arg His Ile Leu Ser Val Leu Val Gln
   1 5 10 15
   Asp Val Asp Gly Ile Ile Ser Arg Val Ser Gly Met Phe Thr Arg Arg
   20 25 30
   Ala Phe Asn Leu Val Ser Leu Val Ser Ala Lys Thr Glu Thr His Gly
   35 40 45
   Ile Asn Arg Ile Thr Val Val Val Asp Ala Asp Glu Leu Asn Ile Glu
   50 55 60
   Gln Ile Thr Lys Gln Leu Asn Lys Leu Ile Pro Val Leu Lys Val Val
   65 70 75 80
   Arg Leu Asp Glu Glu Thr Thr Ile Ala Arg Ala Ile Met Leu Val Lys
   85 90 95
   Val Ser Ala Asp Ser Thr Asn Arg Pro Gln Ile Val Asp Ala Ala Asn
   100 105 110
   Ile Phe Arg Ala Arg Val Val Asp Val Ala Pro Asp Ser Val Val Ile
   115 120 125
   Glu Ser Thr Gly Thr Pro Gly Lys Leu Arg Ala Leu Leu Asp Val Met
   130 135 140
   Glu Pro Phe Gly Ile Arg Glu Leu Ile Gln Ser Gly Gln Ile Ala Leu
   145 150 155 160
   Asn Arg Gly Pro Lys Thr Met Ala Pro Ala Lys Ile
   165 170
<210> 11
   <211> 28
   <212> DNA
   <213> Corynebacterium glutamicum
<220>
   <221> repeat_region
   <222> (1)..(28)
   <223> IR1
<400> 11
   ccccgcgcgc cgggtgggga gcgaaggg 28
<210> 12
   <211> 29
   <212> DNA
   <213> Corynebacterium glutamicum
<220>
   <221> repeat_region
   <222> (1)..(29)
   <223> IR2
<400> 12
   gcgattttgc atgttttact caaaattac 29
<210> 13
   <211> 2619
   <212> DNA
   <213> Corynebacterium glutamicum
<220>
   <221> promoter
   <222> (1)..(177)
   <223> PprpD2
<220>
   <221> RBS
   <222> (178)..(206)
   <223> RBS gap
<220>
   <221> misc_feature
   <222> (207)..(209)
   <223> atg-Startcodon
<220>
   <221> gene
   <222> (207)..(2087)
   <223> Kodierregion ilvB
<220>
   <221> gene
   <222> (2101)..(2619)
   <223> Kodierregion ilvN
<400> 13
   ctccagcgtc cacgaatatg cccccgcgcg ccgggtgggg agcgaaggga acccccaagg 60
   aattggcgtt gaggtggcga ttttgcatgt tttactcaaa attactttgg tggtcacaaa 120
   attacacaac ttttacagtg acctagatcg ctttttaaag aattagcgtg gtgtgcatgc 180
   atatttatcg cgagaggaga cacaacatga atgtggcagc ttctcaacag cccactcccg 240
   ccacggttgc aagccgtggt cgatccgccg cccctgagcg gatgacaggt gcacaggcaa 300
   ttgttcgatc gctcgaggag cttaacgccg acatcgtgtt cggtattcct ggtggtgcgg 360
   tgctaccggt gtatgacccg ctctattcct ccacaaaggt gcgccacgtc ctggtgcgcc 420
   acgagcaggg cgcaggccac gcagcaaccg gctacgcgca ggttactgga cgcgttggcg 480
   tctgcattgc aacctctggc ccaggcgcaa ccaacttggt taccccaatc gctgatgcaa 540
   acttggactc cgttcccatg gttgccatca ccggccaggt cggaagtggc ctgctgggta 600
   ccgatgcttt ccaggaagcc gatatccgcg gcatcaccat gccagtgacc aagcacaact 660
   tcatggtcac cgaccccaac gacattccac aggcattggc tgaggcattc cacctcgcga 720
   ttactggtcg ccctggccct gttctggtgg atattcctaa ggatgtccaa aacgctgaat 780
   tggatttcgt ctggccacca aagatcgacc tgccaggcta ccgcccagtt tctactccgc 840
   atgctcgaca gattgagcag gctgtcaaac tgatcggtga agccaaaaag ccagtccttt 900
   acgttggtgg tggcgtaatc aaggctgacg cacacgaaga gcttcgtgcg ttcgctgagt 960
   acaccggcat cccagttgtc accaccttga tggctttggg tactttccca gagtctcacg 1020
   agctgcacat gggtatgcca ggcatgcatg gcactgtgtc cgctgttggt gcactgcagc 1080
   gcagcgacct gctgattgct atcggctccc gctttgatga ccgcgtcacc ggtgacgttg 1140
   acaccttcgc gcctgacgcc aagatcattc acgccgacat tgatcctgcc gaaatcggca 1200
   agatcaagca ggttgaggtt ccaatcgtgg gcgatgcccg cgaagttctt gctcgtctgc 1260
   tggaaaccac caaggcaagc aaggcagaga ccgaggacat ctccgagtgg gttgactacc 1320
   tcaagggcct caaggcacgt ttcccgcgtg gctacgacga gcagccaggc gatctgctgg 1380
   caccacagtt tgtcattgaa accctgtcca aggaagttgg ccccgacgca atttactgcg 1440
   ccggcgttgg ccagcaccaa atgtgggcag ctcagttcgt tgactttgaa aagccacgca 1500
   cctggctcaa ctccggtgga ctgggcacca tgggctacgc agttcctgcg gcccttggag 1560
   caaaggctgg cgcacctgac aaggaagtct gggctatcga cggcgacggc tgtttccaga 1620
   tgaccaacca ggaactcacc accgccgcag ttgaaggttt ccccattaag atcgcactaa 1680
   tcaacaacgg aaacctgggc atggttcgcc aatggcagac cctattctat gaaggacggt 1740
   actcaaatac taaacttcgt aaccagggcg agtacatgcc cgactttgtt accctttctg 1800
   agggacttgg ctgtgttgcc atccgcgtca ccaaagcgga ggaagtactg ccagccatcc 1860
   aaaaggctcg agagatcaac gaccgcccag tagtcatcga cttcatcgtc ggtgaagacg 1920
   cacaggtatg gccaatggtg tctgctggat catccaactc cgatatccag tacgcactcg 1980
   gattgcgccc attctttgat ggtgatgaat ctgcagcaga agatcctgcc gacattcacg 2040
   aagccgtcag cgacattgat gccgccgttg aatcgaccga ggcataagga gagacccaag 2100
   atggctaatt ctgacgtcac ccgccacatc ctgtccgtac tcgttcagga cgtagacgga 2160
   atcatttccc gcgtatcagg tatgttcacc cgacgcgcat tcaacctcgt gtccctcgtg 2220
   tctgcaaaga ccgaaacaca cggcatcaac cgcatcacgg ttgttgtcga cgccgacgag 2280
   ctcaacattg agcagatcac caagcagctc aacaagctga tccccgtgct caaagtcgtg 2340
   cgacttgatg aagagaccac tatcgcccgc gcaatcatgc tggttaaggt ctctgcggac 2400
   agcaccaacc gtccgcagat cgtcgacgcc gcgaacatct tccgcgcccg agtcgtcgac 2460
   gtggctccag actctgtggt tattgaatcc acaggcaccc caggcaagct ccgcgcactg 2520
   cttgacgtga tggaaccatt cggaatccgc gaactgatcc aatccggaca gattgcactc 2580
   aaccgcggtc cgaagaccat ggctccggcc aagatctaa 2619
<210> 14
   <211> 1421
   <212> DNA
   <213> Corynebacterium glutamicum
<220>
   <221> gene
   <222> (7)..(636)
   <223> 3'-Bereich des ilvB-Gens
<220>
   <221> gene
   <222> (650)..(1168)
   <223> ilvN-Gen
<220>
   <221> misc_feature
   <222> (707)..(715)
   <223> mutierter Genabschnitt
<400> 14
   gaattccaga tgtgggcagc tcagttcgtt gactttgaaa agccacgcac ctggctcaac      60
   tctggcggcc tgggcaccat gggctacgca gttcctgcgg ctcttggagc aaaggctggc     120
   gcacctgaca aggaagtctg ggctatcgac ggcgacggct gtttccagat gaccaaccag     180
   gaactcacca ccgccgcagt tgaaggtttc cccattaaga tcgcactaat caacaacgga     240
   aacctgggca tggttcgcca atggcagacc ctattctatg aaggacggta ctcaaatact     300
   aaacttcgta accagggcga gtacatgccc gactttgtta ccctttctga gggacttggc     360
   tgtgttgcca tccgcgtcac caaagcggag gaagtactgc cagccatcca aaaggctcga     420
   gagatcaacg accgcccagt agtcatcgac ttcatcgtcg gtgaagacgc acaggtatgg     480
   ccaatggtgt ctgctggatc atccaactcc gatatccagt acgcactcgg attgcgccca     540
   ttctttgatg gtgatgaatc tgcagcagaa gatcctgccg acattcacga agccgtcagc     600
   gacattgatg ccgccgttga atcgaccgag gcataaggag agacccaaga tggctaattc     660
   tgacgtcacc cgccacatcc tgtccgtact cgttcaggac gtagacgatg acttttcccg     720
   cgtatcaggt atgttcaccc gacgcgcatt caacctcgtg tccctcgtgt ctgcaaagac     780
   cgaaacactc ggcatcaacc gcatcacggt tgttgtcgac gccgacgagc tcaacattga     840
   gcagatcacc aagcagctca acaagctgat ccccgtgctc aaagtcgtgc gacttgatga     900
   agagaccacc atcgcccgcg caatcatgct ggttaaggtc tctgcggata gcaccaaccg     960
   tccgcagatc gtcgacgccg cgaacatctt ccgcgcccga gtcgtcgacg tggctccaga    1020
   ctctgtggtt attgaatcca caggcacccc aggcaagctc cgcgcactgc ttgatgtgat    1080
   ggaaccattc ggaatccgcg aactgatcca atccggacag attgcactca accgcggtcc    1140
   gaagaccatg gctccggcca agatctaaac agcaattaat ctgattgcac ctgctgcata    1200
   aatgtgacta gtcaaacacc gtctaattgc atgtgtgtgg tagaacaata atgtagttgt    1260
   ctgcccaagc gagtttaact cccacgattt acagtggggg cagacatctt ttcaccaaaa    1320
   tttttacgaa aggcgagatt ttctcccatg gctattgaac tgctttatga tgctgacgct    1380
   gacctctcct tgatccaggg ccgcaaggtt gccataagct t                        1421
<210> 15
   <211> 19
   <212> DNA
   <213> Corynebacterium glutamicum
<220>
   <221> misc_feature
   <222> (1)..(19)
   <223> Testprimer 3
<400> 15
   cccagtagtc atcgacttc 19
<210> 16
   <211> 20
   <212> DNA
   <213> Corynebacterium glutamicum
<220>
   <221> misc_feature
   <222> (1)..(20)
   <223> Testprimer 4
<400> 16
   cagcgtcagc atcataaagc 20

## Beschreibung

Gegenstand der Erfindung ist ein Verfahren zur Herstellung von L-Valin unter Verwendung von Mikroorganismen, in denen ein durch Propionat induzierbarer Promotor die regulierte Expression der Gene ilvBN ermöglicht.

### Stand der Technik

Aminosäuren und Ketosäuren finden in der Humanmedizin, in der pharmazeutischen Industrie, in der Kosmetik, in der Lebensmittelindustrie und in der Tierernährung Anwendung.

Zahlreiche dieser Verbindungen werden durch Fermentation von Stämmen coryneformer Bakterien, insbesondere Corynebacterium glutamicum, hergestellt. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Valin-Analogon 2-Thiazolalanin oder das Leucin-Analogon 4-Azaleucin oder 5,5,5- Trifluorleucin sind und chemische Verbindungen, beispielsweise die L-Aminosäuren L-Valin oder L-Leucin, produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man zum Beispiel einzelne Aminosäure-Biosynthesegene verstärkt oder abschwächt, z.B. auch mit zeitlicher Regulation der Genexpression im Verlauf der Produktion, und die Auswirkung auf die Produktion der chemischen Verbindung untersucht.

Zusammenfassende Darstellungen zur Biologie, Genetik und Biotechnologie von Corynebacterium glutamicum sind im "Handbook of Corynebacterium glutamicum" (Eds.: L. Eggeling und M. Bott, CRC Press, Taylor & Francis, 2005), in der Spezialausgabe des Journal of Biotechnolgy (Chief Editor: A. Pühler) mit dem Titel "A New Era in Corynebacterium glutamicum Biotechnology" (Journal of Biotechnolgy 104/1-3, (2003)) und im Buch von T. Scheper (Managing Editor) "Microbial Production of L-Amino Acids" (Advances in Biochemical Engineering/Biotechnology 79, Springer Verlag, Berlin, Deutschland, 2003) zu finden.

Die Nukleotidsequenz des Genoms von Corynebacterium glutamicum ist bei Ikeda und Nakagawa (Applied Microbiology and Biotechnology 62, 99-109 (2003)), in der EP 1 108 790 und bei Kalinowski et al. (Journal of Biotechnolgy 104/1-3, (2003)) beschrieben.

Die Nukleotidsequenzen des Genoms von Corynebacterium glutamicum sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Grundsätzlich gibt es zwei Möglichkeiten der Expression von Genen. Bei der kontinuierlichen Expression wird das Gen über einen konstitutiven Promoter durchgehend exprimiert und das entsprechende Protein akkumuliert sich in der Zelle.

Dagegen wird bei der induzierten Expression ein induzierbarer Promotor verwendet. Durch einen Induktor wird die Expression des Zielgens induziert, also freigeschaltet. Diese Methode wird verwendet, wenn die (Über)expression negative Auswirkungen auf den Produktionsorganismus hat. Ursachen hierfür können eine hohe Belastung der stoffwechselbedingten Ressourcen während der Wachstumsphase sein. Die Folge ist langsameres Wachstum und damit verlängerte Laufzeiten des Bioreaktors und damit verbunden eine Erhöhung der Kosten bei industrieller Produktion. Auch von Vorteil ist eine induzierte Expression bei zelltoxischen Produkten. Hier kommt es nach der Induktion der Expression zu einer Selbstvergiftung und zum Absterben der Zelle. Im Hinblick auf die Wirtschaftlichkeit eines Produktionsprozesses wird deshalb versucht, den Prozess in eine Wachstumsphase und eine Produktionsphase zu unterteilen. In der Wachstumsphase wird eine möglichst große Menge an Biomasse erzeugt und in der Produktionsphase wird dann durch Induktion des Promotors das Zielprotein produziert. Auf diese Weise kann man eine maximale Ausbeute erhalten, wodurch der Prozess deutlich wirtschaftlicher wird.

Für die regulierbaren Escherichia coli-Promotoren lac, Lambda PL und trp konnte bereits gezeigt werden, dass sie in coryneformen Bakterien zur regulierten Expression verschiedener Gene eingesetzt werden können (Tsuchiya und Morinaga, Bio/Technology 6 (1988) 428-431).

Den Idealfall für einen induzierbaren Promotor stellt ein coryneformer Promotor dar, der durch einen leicht verfügbaren, preiswerten Stoff reguliert wird.

EP 0530765 B1 (Kyowa Hakko) beschreibt die Verwendung eines induzierbaren Promotors aus Corynebakterien, hier des Isocitratlyase-Gens, zur Herstellung von Enzymen wie [beta]-Galactosidase, Chloramphenicol Acetyltransferase und ICL sowie physiologisch aktiven Proteinen wie Insulin oder [alpha]-, [beta]- or [gamma]-Interferon. Dieser Promotor führt zur Expression von Genen, solange sich nur eine von Zucker verschiedene Kohlenstoffquelle (C-Quelle) im Medium befindet, bei Anwesenheit von Zucker wird er reprimiert. Da jedoch in gängigen Fermentationsmedien Zucker als C-Quelle eingesetzt werden, wäre es sinnvoll, einen regulierbaren Promotor zu erhalten, der auch in Anwesenheit von Zuckern mit einem preiswerten Induktor zur Expression eines Gens führt.

DE4440118 C1 (wie US5965391, FZ Jülich) beansprucht die Verwendung eines induzierbaren Promotors aus Corynebakterien, hier des Malatsynthase-Gens aceB, zur Herstellung von Proteinen; Induktoren sind hier die Kohlenstoffquellen Lactat, Pyruvat oder Acetat.

Der Promotor des prpDBC2-Operons von Corynebacterium glutamicum, dessen Gene für das Wachstum auf Propionat als Haupt-C-Quelle essentiell sind und für die Enzyme 2-Methylcitrat Dehydratase (PrpD2), 2-Methylisocitrat Lyase (PrpB2) and 2-Methylcitrat Synthase (PrpC2) kodieren, wurde von Plassmeier et al. (Journal of Biotechnology 159/1-2 (2012)) beschrieben. Analysen von Promotor-Testvektor-Konstrukten führten zur Identifizierung einer 121 Basenpaaren langen Operator-Region oberhalb des prpDBC2-Operons, die für eine Propionat-induzierte Transkription durch den Aktivator PrpR erforderlich ist. EMSA Studien ergaben, dass 2-Methylcitrat wahrscheinlich als Coaktivator des PrpR fungiert.

Das Dokument PLASSMEIER, J.K. ET AL. "A propionate-inducible expression system based on the Corynebacterium gtutamicum prpD2 promoter and PrpR activator and its application for the redirection of amino acid biosynthesis pathways" (JOURNAL OF BIOTECHNOLOGY, Bd. 163, Nr. 2, 20. Januar 2013, Seiten 225-232) offenbart ein Verfahren zur Herstellung von L-Isoleucin durch Fermentation von Mikroorganismen der Gattung Corynebacterium, enthaltend ein Polynukleotid mit Operatoraktivität, dessen Sequenz zu mindestens 85% identisch ist mit der Sequenz von Position 1 bis 121 gemäß SEQ ID NO: 1, 2 oder 3, an welches der Aktivator PrpR bindet, und dem am 3'-Ende ein zweites Polynukleotid mit Promotoraktivität sowie das für die Homoserin Dehydrogenase kodierende Gen hom funktionsfähig nachgeschaltet ist und das die Transkription des Gens hom in Abhängigkeit von der Anlagerung des Aktivators PrpR reguliert, in einem Medium, dem als Induktor Propionat zugegeben wird, worauf L-Isoleucin synthetisiert wird, unter Bedingungen, bei denen L-Isoleucin im Medium angereichert wird.

Das Dokument EP 1 096 010 A1 (DEGUSSA AG; FORSCHUNGSZENTRUM JÜLICH GMBH, 2. Mai 2001) offenbart ein Verfahren zur Herstellung von L-Valin durch Fermentation von Mikroorganismen der Gattung Corynebacterium, enthaltend ein Polynukleotid mit Promotoraktivität sowie die für die Untereinheiten einer Acetolactat-Synthase kodierenden Gene ilvB und ilvN, in einem Medium unter Bedingungen, bei denen L-Valin im Medium und/oder in den Zellen angereichert wird.

Das Dokument DE 101 10 344 A1 (DEGUSSA AG, 16. Mai 2002) offenbart die Tatsache, dass für die Produktion von L-Valin unter anderem die Expression der für die Untereinheiten einer Acetolactat-Synthase kodierenden Gene ilvB und ilvN und/oder des für die Homoserin Dehydrogenase kodierende Gens hom verstärkt werden sollte.

### Aufgabe der Erfindung

Der Erfindung lag die Aufgabe zugrunde, ein neues Verfahren zur Herstellung der L-Aminosäure L-Valin mit vorzugsweise verbesserter Ausbeute und/oder höherer Endkonzentration des Produktes intrazellulär und/oder im Medium zur Verfügung zu stellen. Es sollte hierbei vorzugsweise eine Verbesserung der spezifischen Ausbeute (d.h. Ausbeute an gewünschtem Produkt im Verhältnis zu eingesetzter Kohlenstoffquelle) vorliegen.

Das neue Verfahren sollte es hierbei vorzugsweise ermöglichen, die Produktion von L-Valin unabhängig von der Hauptkohlenstoffquelle des Mediums zu regulieren.

Bei dem neuen Verfahren sollte außerdem vorzugsweise die Bildung von unerwünschten Nebenprodukten, insbesondere die Bildung des unerwünschten Nebenprodukts Alanin, möglichst unterbunden werden, da die Abtrennung des Nebenproduktes sehr aufwendig und kostenintensiv ist und sich außerdem negativ auf die Produktreinheit der Brühe und die Kohlenstoffausbeute auswirkt.

Das eingesetzte Verfahren sollte außerdem vorzugsweise zu einer Erhöhung der genetischen Stabilität des für die Produktion eingesetzten Stammes führen und damit eine hohe Anzahl an Generationen im Fermentationsprozeß (Anzuchtstufen und Hauptfermenter) ohne Abfall der Leistungdaten ermöglichen.

### Beschreibung der Erfindung

Gelöst wird die erfindungsgemäße Aufgabe durch die Verwendung eines Operators, an welchen der Aktivator PrpR bindet, zur Regulation der Expression der Gene ilvBN.

Bei ilvBN (EC-Nr. 4.1.3.18) handelt es sich um die für die Untereinheiten einer Acetolactat-Synthase kodierenden Gene.

Gegenstand der Erfindung ist daher ein Verfahren zur Herstellung von L-Valin durch Fermentation von Mikroorganismen der Gattung Corynebacterium, enthaltend in replizierbarer Form ein Polynukleotid mit Operatoraktivität, dessen Sequenz zu mindestens 85 %, vorzugsweise zu mindestens 90, 92, 94 oder 96 %, insbesondere zu mindestens 97, 98 oder 99 %, besonders bevorzugt zu 100 % identisch ist zu der Sequenz von Position 1 bis 121 gemäß SEQ ID NO: 1, 2 oder 3, an welches der Aktivator PrpR bindet und dem am 3'-Ende ein zweites Polynukleotid mit Promotoraktivität sowie die für die Untereinheiten einer Acetolactat-Synthase kodierenden Gene ilvB und ilvN funktionsfähig nachgeschaltet sind und das die Transkription der Gene ilvBN in Abhängigkeit von der Anlagerung des Aktivators PrpR, der durch den Co-Aktivator 2-Methylcitrat aktiviert wird, reguliert,

in einem Medium, dem nach einer ersten Phase ohne Induktor in einer darauf folgenden zweiten Phase als Induktor Propionat oder 2-Methylcitrat zugegeben wird, worauf die ilvBN-Gene exprimiert werden und somit die gewünschte L-Aminosäure oder α-Ketosäure synthetisiert wird, unter Bedingungen, bei denen die gewünschte L-Aminosäure oder α-Ketosäure im Medium oder in den Zellen angereichert wird.

Bei dem Polynukleotid mit Operatoraktivität handelt es sich um einen Operator, der natürlicherweise die Expression einer 2-Methylcitrat-Dehydratase in coryneformen Bakterien reguliert oder um ein von einem solchen Operator abgeleitetes Polynukleotid.

Das Polynukleotid mit Operatoraktivität umfasst vorzugsweise ein Polynukleotid, dessen Sequenz zu mindestens 90 %, vorzugsweise zu mindestens 92, 94 oder 96 %, insbesondere zu mindestens 97, 98 oder 99 %, besonders bevorzugt zu 100 % identisch ist zu der Sequenz gemäß SEQ ID NO: 11 oder zu der Sequenz von Position 22 bis Position 49 gemäß SEQ ID NO: 1, 2 oder 3 (im Folgenden auch "IR 1" genannt) sowie ein Polynukleotid, dessen Sequenz zu mindestens 90 %, vorzugsweise zu mindestens 92, 94 oder 96 %, insbesondere zu mindestens 97, 98 oder 99 %, besonders bevorzugt zu 100 % identisch ist zu der Sequenz gemäß SEQ ID NO: 12 oder zu der Sequenz von Position 77 bis Position 105 gemäß SEQ ID NO: 1, 2 oder 3 (im Folgenden auch "IR 2" genannt). Die Sequenz "IR 1" ist hierbei in einer bevorzugten Ausführungsform in dem Polynukleotid mit Operatoraktivität an Position 22 bis 49 angeordnet, während die Sequenz "IR 2" in einer bevorzugten Ausführungsform an Position 77 bis 105 angeordnet ist.

In einer erfindungsgemäß bevorzugten Ausführungsform ist das Polynukleotid mit Operatoraktivität Teil eines längeren Polynukleotids, vorzugsweise eines Polynukleotids mit einer Sequenzidentität von mindestens 90 %, vorzugsweise von mindestens 92, 94 oder 96 %, insbesondere von mindestens 97, 98 oder 99 %, besonders bevorzugt von 100 %, zu der Sequenz von Position 1 bis 177 gemäß SEQ ID NO: 1, 2 oder 3.

Bei dem Gen ilvB handelt es sich vorzugsweise um ein Polynukleotid kodierend für ein Polypeptid mit einer Aminosäuresequenz, die eine Identität von mindestens 90 %, vorzugsweise von mindestens 92, 94 oder 96 %, insbesondere von mindestens 97, 98 oder 99 %, besonders bevorzugt von 100 %, zu der Aminsoäuresequenz gemäß SEQ ID NO: 9 aufweist.

Besonders bevorzugt handelt es sich hierbei um ein Polynukleotid, dessen Sequenz zu mindestens 90 %, vorzugsweise zu mindestens 92, 94 oder 96 %, insbesondere zu mindestens 97, 98 oder 99 %, besonders bevorzugt zu 100 %, identisch ist zu der Sequenz von Position 499 bis 2379 gemäß SEQ ID NO: 8.

Bei dem Gen ilvN handelt es sich vorzugsweise um ein Polynukleotid kodierend für ein Polypeptid mit einer Aminosäuresequenz, die eine Identität von mindestens 90 %, vorzugsweise von mindestens 92, 94 oder 96 %, insbesondere von mindestens 97, 98 oder 99 %, besonders bevorzugt von 100 %, zu der Aminosäuresequenz gemäß SEQ ID NO: 10 aufweist.

Besonders bevorzugt handelt es sich hierbei um ein Polynukleotid, dessen Sequenz zu mindestens 90 %, vorzugsweise zu mindestens 92, 94 oder 96 %, insbesondere zu mindestens 97, 98 oder 99 %, besonders bevorzugt zu 100 %, identisch ist zu der Sequenz von Position 2393 bis 2911 gemäß SEQ ID NO: 8.

Die durch die Gene ilvB und ilvN kodierten Polypeptide lagern sich zu einer funktionellen Acetolactat-Synthase zusammen.

Zur Induktion der Expression wird vorzugsweise Propionat bzw. Propionsäure verwendet. Insofern findet eine "Propionat-Induktion" statt. Das Propionat wird in vitro in den eigentlichen Co-Aktivator, das 2-Methylcitrat, umgewandelt. Alternativ kann auch 2-Methylcitrat direkt zur Induktion verwendet werden, wird jedoch wegen der geringeren Verfügbarkeit weniger bevorzugt eingesetzt. Erfindungsgemäß umfasst der Begriff "Propionat-Induktion" auch die Induktion mit 2-Methylcitrat.

Nach Beendigung der Produktion wird L-Valin vorzugsweise isoliert, wobei ggf. weitere Bestandteile der Fermentationsbrühe und/oder die Biomasse in ihrer Gesamtheit oder Anteilen (> 0 bis 100%) im isolierten Produkt verbleiben oder vollständig entfernt werden.

Im erfindungsgemäßen Verfahren erfolgt zuerst eine induktionsfreie Anzuchtphase (Wachstumsphase, erste Phase) zur Bereitstellung von Biomasse. In dieser Phase wird vorzugsweise kein bzw. kaum L-Valin gebildet (< 5 g/l). In der sich anschließenden Produktionsphase (Induktionsphase, zweite Phase) wird die Produktion durch Induktion der Biosynthesegene ilvBN mittels Propionat oder 2-Methylcitrat induziert. Die Anzuchtphase beinhaltet alle Kultivierungsschritte, angefangen vom Rückstellmuster, über vorzugsweise eingesetzte Schüttelkolbenstufen bis hin zum vorzugsweise eingesetzten Anzuchtfermenter. Die Anzuchtphase kann auch noch die erste Phase der Hauptfermentation umfassen. Bevorzugt ist die Anzuchtphase spätestens nach den ersten 3-15 Stunden, vorzugsweise 5-10 Stunden, der Hauptfermentation abgeschlossen.

Die Induktionsphase umfasst bevorzugt die Zeit von 0-20 Stunden nach Animpfen des Hauptfermenters bis zum Ende der Hauptfermentation. Eine Induktion zu einem früheren Zeitpunkt, also im Anzuchtfermenter, kann vorteilhaft sein und stellt eine besondere Ausführungsform des erfindungsgemäßen Verfahrens dar.

Eine genaue Regulierung/Dosierung der Propionsäurekonzentration in der Produktionsphase ist notwendig, um einerseits die Induktion der Aminosäure- bzw. Ketosäuresynthese aufrecht zu erhalten und andererseits die toxische Wirkung des Propionats bzw. eines seiner Abbau-Intermediate (Propionyl-CoA, 2-Methylcitrat) zu verhindern. Die bevorzugte Propionsäurekonzentration in der Induktionsphase liegt im Bereich von 0,1 - 10 g/l. Die Propionsäure kann kontinuierlich in Form eines Propionsäurefeeds oder diskontinuierlich in Form von einem oder mehreren Propionsäurepulsen zu verschiedenen Zeitpunkten während der Induktionsphase zugegeben werden. Die einmalige Dosierung der Propionsäure als Medienbestandteil des Fermentationshauptmediums ist ebenfalls möglich und stellt eine besondere Auführungsform des erfindungsgemäßen Verfahrens dar.

Ein großes Problem bei der Herstellung von L-Valin stellt normalerweise die Bildung von Nebenprodukten dar. Die gebildeten Nebenprodukte vermindern die Kohlenstoffausbeute und müssen zudem gegebenenfalls abgetrennt werden, was sehr aufwendig und kostenintensiv ist.

Ein Beispiel für die Bildung eines Nebenproduktes stellt die Bildung des Nebenproduktes Alanin bei der Valin-Biosynthese dar. Die Bildung von Alanin nimmt bei den herkömmlichen Produktions-Prozessen, in denen gewöhnlich eine konstitutive Expression stattfindet, in Abhängigkeit von der Anzahl der Zell-Generationen zu.

Vom Rückstellmuster bis zur Ernte des Produktionsfermenters werden bei einem dreistufigen Verfahren (Schüttelkolben, Anzuchtfermenter, Hauptfermenter) etwa 20-25 Generationen durchlaufen, bei einem vierstufigen Verfahren (Schüttelkolben, PreSeed-Fermenter, Anzuchtfermenter, Hauptfermenter) werden sogar über 30 Generationen durchlaufen. Beim Durchlaufen derart vieler Generationen ist die Nebenproduktbildung und Biomassenbildung normalerweise entsprechend stark erhöht.

Erfindungsgemäß wurde hingegen eine sehr stark reduzierte Nebenproduktbildung und Biomassenbildung festgestellt. Außerdem wurde festgestellt, dass die Nebenproduktbildung und Biomassenbildung bei erfindungsgemäßen Produktionsverfahren unabhängig von der Anzahl der durchlaufenen Generationen ist. Dies lässt darauf schließen, dass die erfindungsgemäße Verfahrensführung zu einer erhöhten genetischen Stabilität des eingesetzten Stammes führt. Die Anzuchtphase kann daher prinzipiell beliebig verlängert werden, was für die Verfahrensführung besonders vorteilhaft ist.

Ein erfindungsgemäß besonders bevorzugtes Verfahren zeichnet sich daher dadurch aus, dass es mindestens vier Stufen, nämlich mindestens drei Anzuchtstufen und eine Produktionsstufe, umfasst. Die Anzucht umfasst hierbei vorzugsweise die Anzucht im Schüttelkolben, im PreSeed-Fermenter sowie im Seed-Fermenter. Die Produktion erfolgt vorzugsweise in einem Produktionsfermenter.

Ein weiteres erfindungsgemäß besonders bevorzugtes Verfahren zeichnet sich daher dadurch aus, dass die eingesetzten Bakterien während der Anzuchtphase mindestens 16, vorzugsweise mindestens 24, Generationen durchlaufen und/oder während der gesamten Fermentation (inklusive Anzucht und Produktion) mindestens 25, vorzugsweise mindestens 30, Generationen durchlaufen.

Offenbart ist auch die Verwendung eines Polynukleotids mit Operatoraktivität, an welches der Aktivator PrpR bindet, wobei das Polynukleotid eine Sequenz besitzt, die zu mindestens 85%, 90%, 92%, 94%, 96%, 97%, 98%, 99% oder 100%, bevorzugt zu mindestens 97%, besonders bevorzugt zu mindestens 98%, ganz besonders bevorzugt zu mindestens 99% und äußerst bevorzugt 100% identisch ist zu der Sequenz von Position 1 bis 121 gemäß SEQ ID NO: 1, 2 oder 3, zur Regulation der Expression der Gene ilvBN, vorzugsweise in Kombination mit einem den Genen vorgeschalteten Promotor.

Offenbart ist auch eine Expressionskassette, umfassend ein Polynukleotid mit Operatoraktivität, dessen Sequenz zu mindestens 85%, 90%, 92%, 94%, 96%, 97%, 98%, 99% oder 100%, bevorzugt zu mindestens 97%, besonders bevorzugt zu mindestens 98%, ganz besonders bevorzugt zu mindestens 99% und äußerst bevorzugt 100% identisch ist zu der Sequenz von Position 1 bis 121 gemäß SEQ ID NO:1, 2 oder 3, einen nachgeschalteten Promotor sowie die für eine Acetolactat-Synthase kodierenden Gene ilvBN.

Das Polynukleotid mit Operatoraktivität weist hierbei vorzugsweise stets dic zuvor als bevorzugt hervorgehobenen Eigenschaften auf, insbesondere die konservierten Regionen "IR 1" und "IR 2".

Bei dem den ilvBN-Genen vorgeschalteten Promotor bzw. dem dem Operator nachgeschalteten Promotor kann es sich um jeden beliebigen Promotor handeln.

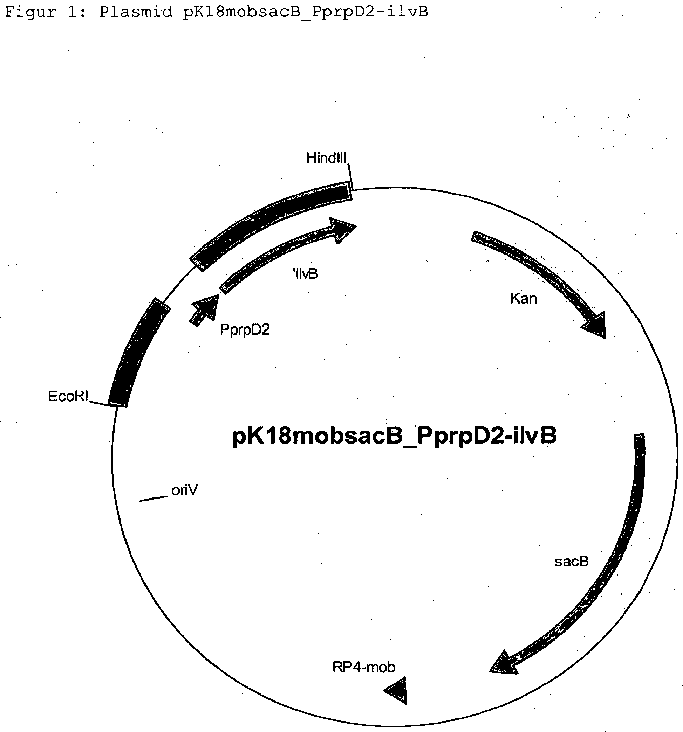

Beispiele für erfindungsgemäß bevorzugt in Corynebacterium glutamicum einsetzbare Promotoren sind beispielsweise in Fig. 1 des Übersichtsartikels von Patek et al. (Journal of Biotechnology 104(1-3), 311-323 (2003)) beschrieben. In gleicher Weise können die von Vasicova et al (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden.

In einer bevorzugten Ausführungsform handelt es sich bei dem erfindungsgemäß eingesetzten Promotor um ein Polynukleotid mit Promotoraktivität, dessen Sequenz zu mindestens 90%, 92%, 94%, 96%, 97%, 98%, 99% oder 100%, bevorzugt mindestens 97%, besonders bevorzugt mindestens 98%, ganz besonders bevorzugt mindestens 99% und äußerst bevorzugt 100% identisch ist zu der Sequenz von Position 122 bis 206 gemäß SEQ ID NO: 4.

Bei der Expressionskassette handelt es sich vorzugsweise um eine Expressionskassette mit einer Sequenz gemäß SEQ ID NO: 13.

Weiteroffenbart ist ein Vektor, der eine anmeldungsgemäß offenbarte Expressionskassette enthält.

Kirchner und Tauch (Journal of Biotechnology 104:287-299 (2003)) beschreiben eine Auswahl der in Corynebacterium glutamicum vorzugsweise anzuwendenden Vektoren.

Die homologe Rekombination erlaubt unter Verwendung der anmeldungsgemäß offenbarten Vektoren den Austausch von DNA-Abschnitten auf dem Chromosom gegen anmeldungsgemäß offenbarte Expressionskassetten, welche durch den Vektor in die Zelle transportiert werden. Für die effiziente Rekombination zwischen dem ringförmigen DNA-Molekül des Vektors und der Ziel-DNA auf dem Chromosom wird der auszutauschende DNA-Bereich, der eine anmeldungsgemäß offenbarte Expressionskassette enthält, an den Enden mit Nukleotidsequenzen homolog zum Zielort versehen, wodurch der Ort der Integration des Vektors bzw. des Austauschs der DNA festgelegt wird.

Der Einbau der anmeldungsgemäß offenbarten Expressionskassette kann hierbei am nativen Genort der ilvBN-Gene erfolgen, wobei hierbei vorzugsweise die nativen ilvBN-Gene und gegebenenfalls auch der native Promotor der ilvBN-Gene durch eine anmeldungsgemäß offenbarte Expressionskassette ersetzt wird.

Alternativ kann eine anmeldungsgemäß offenbarte Expressionskassette auch in einen intergenischen Bereich im Chromosom, der keine kodierende Funktion aufweist, oder an einem anderen

Genort integriert werden, wobei der andere Genort vorzugsweise eine Nukleotidsequenz auf dem Chromosom darstellt, welche für das Wachstum der Zellen und die Produktion der Aminosäure bzw. Ketosäure nicht essentiell ist.

Die anmeldungsgemäß offenbarte Expressionskassette kann erfindungsgemäß in einer bevorzugten Ausführungsform auch in mehrfacher Kopie sowie gegebenenfalls an unterschiedlichen Genorten in das Chromosom eingebaut werden.

Anstelle eine anmeldungsgemäß offenbarte Expressionskassette in das Chromosom einzubauen, kann alternativ auch der anmeldungsgemäß offenbarte verwendete Operator in Kombination mit einem Promotor am nativen Genort der ilvBN-Gene in das Chromosom eingebaut werden, wobei vorzugsweise der native Promotor der ilvBN-Gene gegen das Konstrukt aus Operator und Promotor ersetzt wird.

Anstatt die anmeldungengemäß offenbarte Expressionskassette in das Chromosom einzubauen, kann erfindungsgemäß alternativ natürlich auch ein extrachromosomal replizierender Vektor eingesetzt werden, der eine anmeldungsgemäß offenbarte Expressionskassette enthält.

Weiterer Gegenstand der vorliegenden Erfindung ist ebenfalls ein Mikroorganismus, vorzugsweise ein Corynebacterium, vor allem ein Corynebacterium, das L-Valin produziert, der bzw. das eine anmeldungsgemäß offenbarte Expressionskassette und/oder einen anmeldungsgemäß offenbarten Vektor enthält.

Einzelheiten zur Biochemie bzw. chemischen Struktur von Polynukleotiden, wie sie in Lebewesen, wie beispielsweise Mikroorganismen, vorkommen, findet man unter anderem im Lehrbuch "Biochemie" von Berg et al (Spektrum Akademischer Verlag Heidelberg·Berlin, Deutschland, 2003; ISBN 3-8274-1303-6).

Besteht das Polynukleotid aus Desoxyribonukleotid-Monomeren mit den Nukleobasen bzw. Basen Adenin (A), Guanin (G), Cytosin (C) und Thymin (T) so spricht man von Desoxyribo-Polynukleotiden oder Desoxyribonukleinsäure (DNA). Besteht das Polynukleotid aus Ribonukleotid-Monomeren mit den Nukleobasen bzw. Basen Adenin (A), Guanin (G), Cytosin (C) und Uracil (U) so spricht man von Ribo-Polynukleotiden oder Ribonukleinsäure (RNA). In den genannten Polynukleotiden sind die Monomere durch eine 3'→ 5'-Phosphodiesterbindung miteinander.kovalent verbunden.

Unter einem "Polynukleotid mit Operatoraktivität" oder einem "Operator" wird ein Polynukleotid, bevorzugt Desoxyribo-Polynukleotid, bzw. eine Nukleinsäure, bevorzugt Desoxyribonukleinsäure (DNA) verstanden, das/die in funktioneller Verknüpfung über ein Polynukleotid mit Promotoraktivität mit einem zu transkribierenden Polynukleotid durch Interaktion mit verschiedenen regulatorischen Proteinen (Aktivatoren bzw. Repressoren, welche wiederum in Wechselwirkung mit Liganden oder Effektormolekülen treten) die Transkription dieses Polynukleotids an- oder abschalten.

Unter einem "Polynukleotid mit Promotoraktivität" oder einem "Promotor" wird ein Polynukleotid, bevorzugt Desoxyribo-Polynukleotid, bzw. eine Nukleinsäure, bevorzugt Desoxyribonukleinsäure (DNA) verstanden, das/die in funktioneller Verknüpfung mit einem zu transkribierenden Polynukleotid den Initiationspunkt und die Initiationshäufigkeit der Transkription dieses Polynukleotids festlegt, wodurch die Expressionsstärke des kontrollierten Polynukleotids beeinflußt werden kann.

Aufgrund der doppelsträngigen Struktur der DNA ist der zum Strang des Sequenzprotokolls in SEQ ID NO: 1, 2 oder 3 komplementäre Strang ebenfalls offenbart.

Unter "Transkription" wird der Prozess verstanden, durch den ausgehend von einer DNA-Matrize ein komplementäres RNA-Molekül hergestellt wird. An diesem Prozess sind Proteine wie die RNA-Polymerase, sogenannte Sigma-Faktoren und transkriptionelle Regulatorproteine beteiligt. Die synthetisierte RNA (Messenger-RNA, m-RNA) dient dann als Matrize im Prozess der Translation, der dann zum Polypeptid bzw. Protein führt.

Ein Gen ist chemisch gesehen ein Polynukleotid. Ein Polynukleotid, das ein Protein/Polypeptid kodiert, wird hier synonym zum Begriff "Gen" gebraucht. Demnach werden die beiden Begriffe "Gen" und "Kodierregion" synonym verwendet und gleichermaßen die beiden Begriffe "Protein" und "Polypeptid".

Unter einer "funktionsfähigen Nachschaltung oder Verknüpfung" versteht man in diesem Zusammenhang die sequentielle Anordnung des erfindungsgemässen Polynukleotids mit Operatoraktivität mit einem zweiten Polynukleotid mit Promotoraktivität und mit einem weiteren Oligo- oder Polynukleotid, die zu einer Transkription des weiteren Polynukleotids führt.

Handelt es sich bei dem weiteren Polynukleotid um ein Polynukleotid, das für ein Polypeptid/Protein kodiert, bestehend aus der Kodierregion für ein Polypeptid beginnend mit einem Start-Kodon, einschließlich des Stop-Kodons und ggf. einschließlich eines Transkriptionsterminators, so bedeutet "funktionsfähige Nachschaltung oder Verknüpfung" die sequentielle Anordnung, die zu einer Transkription des weiteren Polynukleotids und der Translation der synthetisierten RNA führt.

Das weitere Polynukleotid kodiert für ein oder mehrere Polypeptid(e). Ein für ein Protein/Polypeptid kodierendes Polynukleotid besteht im wesentlichen aus einem Startkodon, ausgewählt aus der Gruppe ATG, GTG und TTG, bevorzugt ATG oder GTG, besonders bevorzugt ATG, einer proteinkodierenden Sequenz und einem oder mehreren Stop-Kodon(en) ausgewählt aus der Gruppe TAA, TAG und TGA.

Kodiert das weitere Polynukleotid für mehrere Proteine/Polypeptide, so kann sich vor jedem Gen eine Ribosomenbindestelle befinden. Hinter dem letzten Gen befindet sich ggf. ein Terminator.

Das weitere Polynukleotid besteht erfindungsgemäß aus den Genen ilvB und ilvN, die für die Untereinheiten einer Acetolactat-Synthase (IlvBN, EC Nr.: 4.1.3.18) kodieren.

Offenbart ist weiterhin die Verwendung der anmeldungsgemäß offenbarten Expressionskassette oder des anmeldungsgemäß offenbarten Vektors zur Expression der ilvBN-Gene in Mikroorganismen. Die anmeldungsgemäß offenbarte Expressionskassette gewährleistet die Transkription und die Translation der synthetisierten RNA, bevorzugt mRNA, zu Polypeptiden, nämlich der beiden Untereinheiten einer Acetolactat-Synthase.

Unter Verwendung der anmeldungsgemäß offenbarten Expressionskassette können zu einem gewünschten Zeitpunkt die Gene ilvBN in Mikroorganismen exprimiert bzw. überexprimiert werden.

Unter Überexpression versteht man allgemein eine Erhöhung der intrazellulären Konzentration oder Aktivität einer Ribonukleinsäure, eines Proteins (Polypeptids) oder eines Enzyms im Vergleich zum Ausgangsstamm (Elternstamm) oder Wildtypstamm, wenn dies der Ausgangsstamm ist. Unter einem Ausgangsstamm (Elternstamm) versteht man den Stamm, an dem die zur Überexpression führende Maßnahme durchgeführt wurde.

Bei der Überexpression werden die Methoden der rekombinanten Überexpression bevorzugt. Hierunter werden alle Methoden zusammengefasst bei denen ein Mikroorganismus unter Verwendung eines in-vitro bereitgestellten DNA-Moleküls hergestellt wird. Derartige DNA-Moleküle umfassen beispielsweise Promotoren, Expressionskassetten, Gene, Allele, Kodierregionen etc.. Diese werden durch Methoden der Transformation, Konjugation, Transduktion oder gleichartige Methoden in den gewünschten Mikroorganismus überführt.

Durch die Maßnahmen der Überexpression unter Verwendung des erfindungsgemäß einzusetzenden Operators und/oder unter Verwendung der anmeldungsgemäß offenbarten Expressionskassette wird die Aktivität oder Konzentration der Acetolactat-Synthase im Allgemeinen vorzugsweise um mindestens 10%, 25%, 50%, 75%, 100%, 150%, 200%, 300%, 400% oder 500%, bevorzugt maximal bis 1000%, 2000%, 4000%, 10000% oder 20000%, bezogen auf die Aktivität oder Konzentration des Polypeptids im Stamm vor der zur Überexpression führenden Maßnahme, erhöht.

Das Ausmaß der Expression beziehungsweise Überexpression kann durch Messung der Menge der vom Gen transkribierten mRNA, durch Bestimmung der Menge des Polypeptids und durch Bestimmung der Enzymaktivität festgestellt werden.

Für die Bestimmung der Menge an mRNA können unter anderem die Methode des "Northern Blotting's" und der quantitativen RT-PCR verwendet werden. Bei der quantitativen RT-PCR wird der Polymerase-Kettenreaktion eine reverse Transkription vorgeschaltet. Hierzu kann das LightCycler™ System der Firma Roche Diagnostics (Boehringer Mannheim GmbH, Roche Molecular Biochemicals, Mannheim, Deutschland) verwendet werden, wie beispielsweise bei Jungwirth et al. (FEMS Microbiology Letters 281, 190-197 (2008)) beschrieben. Die Konzentration des Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden. Die statistische Signifikanz der erhobenen Daten wird mittels eines T-Tests (Gosset, Biometrika 6(1): 1-25 (1908)) ermittelt.

Die Maßnahme zur Überexpression der ilvBN-Gene unter Verwendung des erfindungsgemäß einzusetzenden Operators kann in geeigneter Weise mit weiteren Maßnahmen zur Überexpression kombiniert werden.

Zur Erzielung einer Überexpression stehen im Stand der Technik eine Vielzahl von Methoden zur Verfügung. Hierzu gehört neben der Modifikation der Nukleotidsequenzen, die die Expression des Gens steuern bzw. kontrollieren, auch die Erhöhung der Kopienzahl.

Die Erhöhung der Kopienzahl kann durch Plasmide erfolgen, die im Cytoplasma des Mikroorganismus replizieren. Hierzu sind im Stand der Technik eine Fülle von Plasmiden für die verschiedensten Gruppen von Mikroorganismen beschrieben, mit denen man die gewünschte Erhöhung der Kopienzahl des Gens einstellen kann. Geeignete Plasmide für die Gattung Corynebacterium sind beispielsweise bei Tauch et al. (Journal of Biotechnology 104 (1-3), 27-40, (2003)), oder bei Stansen et al. (Applied and Environmental Microbiology 71, 5920-5928 (2005)) beschrieben.

Die Erhöhung der Kopienzahl um mindestens eine (1) Kopie kann weiterhin durch Einfügen weiterer Kopien in das Chromosom des Mikroorganismus erfolgen. Geeignete Methoden für die Gattung Corynebacterium sind beispielsweise in der Patentschrift WO 03/014330, WO 03/040373 und WO 04/069996 beschrieben.

Die Erhöhung der Genexpression kann weiterhin dadurch erfolgen, dass man mehrere Promotoren vor das gewünschte Gen positioniert bzw. funktionell mit dem zu exprimierenden Gen verknüpft und auf diese Weise zu einer erhöhten Expression gelangt. Beispiele hierfür werden in der Patentschrift WO 2006/069711 beschrieben.

Die Geschwindigkeit der Elongation wird durch die Kodon-Verwendung beeinflusst, durch den Gebrauch von Kodons für im Ausgangsstamm häufig vorkommenden t(transfer)-RNAs kann die Translation verstärkt werden. Desweiteren kann der Austausch eines Start-Kodons zu dem in vielen Mikroorganismen (77% in Escherichia coli) am häufigsten vorkommenden Kodon ATG die Translation erheblich verbessern, da auf RNA-Ebene das Kodon AUG zwei- bis dreimal effektiver ist als zum Beispiel die Kodons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Kodons kann optimiert werden, da zusammenwirkende Effekte zwischen dem Start-Kodon und den flankierenden Bereichen beschrieben sind (Stenstrom et al., Gene 273(2):259-65 (2001); Hui et al., EMBO Journal 3(3):623-9 (1984)).

Anleitungen zum Umgang mit DNA, Verdauung und Ligation von DNA, Transformation und Selektion von Transformanten findet man unter anderem in dem bekannten Handbuch von Sambrook et al. "Molecular Cloning: A Laboratory Manual, Second Edition (Cold Spring Harbor Laboratory Press, 1989).

In einer bevorzugten Ausführungsform werden erfindungsgemäß Mikroorganismen eingesetzt, in denen zusätzlich weitere Gene des Biosyntheseweges der gewünschten L-Aminosäure oder α-Ketosäure verstärkt, insbesondere überexprimiert vorliegen.

Im Zusammenhang mit der Herstellung von L-Valin können bevorzugt eines oder mehrere der Gene bzw. Polynukleotide, die für Enzyme der Biosynthese von L-Valin kodieren, ausgewählt aus der Gruppe:
a) Polynukleotid (ilvC-Gen), das für eine Isomeroreductase (IlvC, EC Nr.: 1.1.1.86) kodiert,
b) Polynukleotid (ilvD-Gen), das für eine Dihydroxy-acid Dehydratase (IlvD, EC Nr.: 4.2.1.9) kodiert,
c) Polynukleotid (ilvE-Gen), das für eine Transaminase (IlvE, EC Nr.: 2.6.1.42) kodiert,
d) Polynukleotid (ilvA-Gen) das für eine Threonindehydratase (IlvA, EC Nr.: 4.3.1.19) kodiert,
e) Polynukleotid (hom-Gen) das für eine Homoserindehydrogenase (Hom, EC-Nr.: 1.2.1.11 kodiert
f) Polynukleotid (thrB-Gen), das für eine Homoserinkinase (ThrB, EC-Nr.: 2.7.1.39) kodiert
g) Polynukleotid (thrC-Gen), das für eine Threoninsynthase (ThrC, EC-Nr.: 4.2.3.1) kodiert
h) Polynukleotid (leuA-Gen), das für eine Isopropylmalatsynthase (LeuA, EC-Nr.: 2.3.3.13) kodiert
i) Polynukleotid (leuB-Gen), das für eine Isopropylmalatdehydrogenase (LeuB, EC-Nr.: 1.1.1.85) kodiert
j) Polynukleotid (leuC-Gen), das für die große Untereinheit einer Isopropylmalatisomerase (LeuC, EC-Nr.: 4.2.1.33) kodiert
k) Polynukleotid (leuD-Gen), das für die kleine Untereinheit einer Isopropylmalatisomerase (LeuD, EC-Nr.: 4.2.1.33) kodiert zusätzlich überexprimiert werden, wobei die Gene hom, ilvA, ilvC, ilvD und ilvE für Valin besonders bevorzugt werden.

In einer bevorzugten Ausführungsform werden Mikroorganismen eingesetzt, in denen die Stoffwechselwege zumindest teilweise abgeschwächt sind, die die Bildung von L-Valin verringern.

Da die Produktion von Valin gewünscht ist, können die Stoffwechselwege für Isoleucin (zur Bildung der Vorstufe alpha-Ketobuttersäure: ilvA, thrB, thrC, hom) und/oder für Leucin (leuA, leuB, leuCD) abgeschwächt sein.

Der Begriff "Abschwächung" beschreibt in diesem Zusammenhang die Verringerung oder Ausschaltung der intrazellulären Aktivität eines oder mehrerer Enzyme (Proteine) in einem Bakterium, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert. Die vollständige bzw. die partielle Abschwächung einzelner Zielgene kann z.B. durch vollständige oder partielle Deletion der Gene bzw. durch Einfügen von Punktmutationen im Strukturgen bzw. im Promotorbereich oder in der Ribosomenbindestelle erreicht werden. Eine weitere Methode zur spezifischen Verringerung der Genexpression ist die Antisense-Technik, wobei kurze Oligodesoxynukleotide oder Vektoren zur Synthese längerer Antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371). Die soeben beschriebene Antisense-Technik kann auch durch Verwendung des erfindungsgemäßen Operators/Promotors erfolgen, in dem dieser in "anti-sense"-Orientierung hinter das Zielgen kloniert wird. Nach Zugabe des Induktors Propionat wird die Bildung einer mRNA des Gegenstranges des abzuschwächenden Zielgens induziert. Durch Anlagerung dieser anti-sense-mRNA an die mRNA des Zielgens wird die Expression des Zielgens reduziert. Die regulierte Expression oder Überexpression der Gene ilvBN bzw. Produktion von L-Valin wird in Mikroorganismen der Gattung Corynebacterium durchgeführt. Innerhalb der Gattung Corynebacterium werden Stämme bevorzugt, die auf folgenden Arten beruhen: Corynebacterium efficiens, wobei der Typstamm als DSM44549 hinterlegt ist, Corynebacterium glutamicum, wobei der Typstamm als ATCC13032 hinterlegt ist, und Corynebacterium ammoniagenes, wobei der Typstamm als ATCC6871 hinterlegt ist. Die Art Corynebacterium glutamicum wird ganz besonders bevorzugt.

Einige Vertreter der Art Corynebacterium glutamicum sind im Stand der Technik auch unter anderen Bezeichnungen bekannt. Hierzu gehören beispielsweise: Stamm ATCC13870, der als Corynebacterium acetoacidophilum bezeichnet wurde, Stamm DSM20137, der als Corynebacterium lilium bezeichnet wurde, Stamm ATCC17965, der als Corynebacterium melassecola bezeichnet wurde, Stamm ATCC14067, der als Brevibacterium flavum bezeichnet wurde, Stamm ATCC13869, der als Brevibacterium lactofermentum bezeichnet wurde, und Stamm ATCC14020, der als Brevibacterium divaricatum bezeichnet wurde.

Der Begriff "Micrococcus glutamicus" für Corynebacterium glutamicum war ebenfalls gebräuchlich. Einige Vertreter der Art Corynebacterium efficiens wurden im Stand der Technik auch als Corynebacterium thermoaminogenes bezeichnet, wie zum Beispiel der Stamm FERM BP-1539.

Die für die Maßnahmen eingesetzten Mikrorganismen bzw. Stämme (Ausgangsstämme) besitzen bevorzugt bereits die Fähigkeit, L-Valin in das sie umgebende Nährmedium auszuscheiden und dort zu akkumulieren. Hierfür wird im Folgenden auch der Ausdruck "produzieren" verwendet. Insbesondere besitzen die erfindungsgemäß eingesetzten Stämme vorzugsweise die Fähigkeit nach der Induktion ≥ (mindestens) 0,5 g/l*h, vorzugsweise mindestens 1,0 oder 2,0 g/l*h L-Valin in der Zelle oder im Nährmedium anzureichern bzw. zu akkumulieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist für den Fachmann verständlich, dass man zu einem für die Maßnahmen der Erfindung geeignetem Mikroorganismus auch dadurch gelangen kann, indem man in einem Wildstamm, wie zum Beispiel in dem Corynebacterium glutamicum Typstamm ATCC 13032 oder in dem Stamm ATCC 14067, zunächst einen erfindungsgemäß zu verwendenden Operator zur regulierten Expression der gewünschten Gene einsetzt und anschließend durch weitere, im Stand der Technik beschriebene, genetische Maßnahmen, den Mikrorganismus veranlasst, L-Valin zu produzieren.

Bekannte Vertreter L-Valin produzierender bzw. ausscheidender Stämme coryneformer Bakterien sind beispielsweise: Brevibacterium lactofermentum FERM BP-1763 (beschrieben in US 5,188,948); Brevibacterium lactofermentum FERM BP-3007 (beschrieben in US 5,521,074); Corynebacterium glutamicum FERM BP-3006 (beschrieben in US 5,521,074); und Corynebacterium glutamicum FERM BP-1764 (beschrieben in US 5,188,948).

L-Valin produzierende Mikrorganismen besitzen typischerweise eine feedback-resistente bzw. desensibilisierte Acetolactat-Synthase (AHAS, EC 4.1.3.18). Sie stellt das erste Enzym der parallelen Stoffwechselwege zur Synthese von Isoleucin, Valin und Leucin dar (Umbarger, H. E. 1987. Biosynthesis of the branched-chain amino acids, p. 352-367. In F. C. Neidhardt, J. L. Ingraham, K. B. Low, B. Magasanik, M. Schaechter, and H.E. Umbarger (ed.), Escherichia coli and Salmonella typhimurium: Cellular and Molecular Biology. American Society for Microbiology, Washington, D.C.). Das Holoenzym besteht immer aus 2 großen Untereinheiten und 2 kleinen Untereinheiten. Die große AHAS-Untereinheit bildet die katalytische Domäne und wird von ilvB kodiert, die kleine Untereinheit, welche als regulatorische Domäne fungiert, wird von ilvN kodiert. Unter feedback-resistenten Acetolactat Synthasen versteht man Acetolactat Synthasen, die im Vergleich zur Wildform (Wildtyp) eine geringere Empfindlichkeit gegenüber der Hemmung durch die verzweigtkettigen Aminosäuren Valin, Leucin und Isoleucin bzw. Mischungen dieser aufweisen. Im Falle der Acetolactat-Synthasen der Art Corynebacterium glutamicum sind die Stämme ATCC13032, ATCC14067 (auch als Brevibacterium flavum bekannt) oder ATCC13869 (auch als Brevibacterium lactofermentum bekannt) der geeignete Wildtyp.

Die in Corynebacterium glutamicum für Acetolactat-Synthase kodierenden Gene ilvBN sind beispielsweise von Keilhauer et al. (Journal of Bacteriology 175(17):5595-603 (1993)) oder in der EP1108790 beschrieben. Die Zugangsnummer L09232 (GenBank, NCBI) stellt die Sequenz der Gene dar. Enzymvarianten der AHAS, die nicht mehr der Feedbackhemmung durch die verzweigtkettigen Aminosäuren (Leucin, Valin, Isoleucin) unterliegen, sind beispielsweise bei Mendel et al. (Journal of Molecular Biology 325, 275-284 (2003)), Elisakova et al. (Applied and Environmental Microbiology 71, 207-213 (2005)), Wada et al. (Bioscience Biotechnology & Biochemistry, 72 (11), 2959-2965, (2008)) und in der EP1491634 beschrieben. Bevorzugt werden Varianten einer feedback-resistenten Acetolactat-Synthase, welche einen oder mehrere der folgenden Aminosäureaustausche in der durch ilvN kodierten kleinen Untereinheit tragen, ausgewählt aus der Gruppe: an Position 20 der Aminosäuresequenz L-Aparaginsäure anstelle von Glycin, an Position 21 der Aminosäuresequenz L-Aparaginsäure anstelle von L-Isoleucin, an Position 22 der Aminosäuresequenz L-Phenylalanin anstelle von L-Isoleucin, an Position 42 jede proteinogene Aminosäure ausgenommen L-Alanin, bevorzugt L-Valin, L-Isoleucin und L-Leucin, besonders bevorzugt L-Valin und gegebenenfalls an Position 47 L-Leucin anstelle von L-Histidin (beschrieben in DE 102011118019 A1).

Die vorliegende Erfindung stellt einen Mikroorganismus bereit, der L-Valin produziert, wobei der Mikroorganismus durch die Verwendung des erfindungsgemäß einzusetzenden Operators eine regulierte Expression der für die Acetolactat-Synthase kodierenden Genen ilvBN ermöglicht bzw. aufweist.

Weiterhin stellt die vorliegende Erfindung ein Verfahren bereit zur fermentativen Herstellung von L-Valin umfassend die Schritte:
a) Kultivieren eines erfindungsgemäßen Mikroorganismus der Gattung Corynebacterum in einem geeignetem Medium, wobei eine Fermentationsbrühe erhalten wird, und
b) Anreichern von L-Valin in der Fermentationsbrühe aus a) und/oder in den Zellen des Mikroorganismus.

Dabei ist bevorzugt, dass man aus der L-Valin enthaltenden Fermentationsbrühe L-Valin oder ein flüssiges oder festes Produkt, das L-Valin enthält, gewinnt.

Die hergestellten Mikroorganismen können kontinuierlich - wie beispielsweise in der WO 05/021772 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren) zum Zwecke der Produktion von L-Valin kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Lactose, Fructose, Maltose, Melasse, Saccharose-haltige Lösungen aus der Zuckerrüben- oder Zuckerrohrverarbeitung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäüre, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Ammoniumphosphat, Kaliumdihydrogenphosphat oder Dikaliumhydrogenphosphat oder die entsprechenden Natrium-haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Propionat wird bevorzugt als Salz zum Medium zugegeben, kann aber auch als Propionsäure zugegeben werden. Als Salze der Propionsäure kommen Magnesium-Propionat, Natrium-Propionat, Calcium-Propionat, Ammonium-Propionat, Kalium-Propionat in Frage. Im Medium liegt Propionat als freie Säure bzw. als Propionat-Anion gelöst vor.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak, Ammoniumhydroxid beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 8,5 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe wie zum Beispiel Antibiotika hinzugefügt werden. Die Fermentation wird bevorzugt unter aeroben Bedingungen durchgeführt. Um diese aufrechtzuerhalten, werden Sauerstoff oder Sauerstoff-haltige Gasmischungen, wie zum Beispiel Luft, in die Kultur eingetragen. Die Verwendung von Flüssigkeiten, die mit Wasserstoffperoxid angereichert sind, ist ebenfalls möglich. Die Fermentation unter sauerstofflimitierenden Bedingungen ist eine weitere besondere erfindungsgemäße Ausführungsform. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch- oder fed-batch-Verfahren wird die Kultivierung bevorzugt solange fortgesetzt, bis sich eine für die Maßnahme der Gewinnung von L-Valin ausreichende Menge, gebildet hat. Dieses Ziel wird normalerweise innerhalb von 10 Stunden bis 160 Stunden erreicht. Bei kontinuierlichen Verfahren sind längere Kultivierungszeiten möglich.' Durch die Tätigkeit der Mikroorganismen kommt es zu einer Anreicherung (Akkumuiation) von L-Valin im Fermentationsmedium und/oder in den Zellen der Mikroorganismen.

Beispiele für geeignete Fermentationsmedien finden sich unter anderem in den Patentschriften 5,770,409, US 5,990,350, US 5,275,940, WO 2007/012078, US 5,827,698, WO 2009/043803, US 5,756,345 oder US 7,138,266.

Die Analyse von L-Valin zur Bestimmung der Konzentration zu einem oder mehreren Zeitpunkt (en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandene valin derivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die Trennparameter waren wie folgt: Durchflussrate 0.4 ml /min; Injektionsvolumen der Probe 20 µl; Temperatur 35°C; Die Detektion erfolgt photometrisch bei 215 nm mit UV. Die Retentionszeit der Propionsäure betrug 30.258 min. Der messbare Bereich lag zwischen 0.09 und 7.514 g/l.

Die Leistung der erfindungsgemäßen Verfahren bzw. Fermentationsprozesse bezüglich eines oder mehrerer der Parameter ausgewählt aus der Gruppe der Konzentration (gebildete Verbindung pro Volumen), der Ausbeute (gebildete Verbindung pro verbrauchter Kohlenstoff-Quelle), der Bildung (gebildete Verbindung pro Volumen und Zeit) und der spezifischen Bildung (gebildete Verbindung pro Zelltrockenmasse bzw. Biotrockenmasse und Zeit oder gebildete Verbindung pro Zellprotein und Zeit) oder auch anderer Prozess-Parameter und Kombinationen davon, ist erfindungsgemäß vorzugsweise erhöht um mindestens 0,5%, mindestens 1%, mindestens 1,5% oder mindestens 2% im Vergleich zu Verfahren bzw. Fermentationsprozessen mit Mikroorganismen, in denen die Expressionskassette nicht vorliegt. Dies ist im Rahmen eines großtechnischen Prozesses als sehr wertvoll anzusehen.

Durch die Maßnahmen der Fermentation erhält man eine Fermentationsbrühe, welche L-Valin enthält.

Anschließend erfolgt die Bereitstellung bzw. Herstellung oder Gewinnung eines L-Valin enthaltenden Produktes in flüssiger oder fester Form.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Produktion der gewünschten Verbindung und typischerweise die Vermehrung bzw. Lebensfähigkeit sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) das im Laufe der Fermentation gebildete L-Valin,
c) die gegebenenfalls im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der jeweiligen gewünschten Verbindung erzeugt und gegebenenfalls ausgeschieden werden.

Die Fermentationsbrühe wird dem Kulturgefäß beziehungsweise dem Fermentationsbehälter entnommen, gegebenenfalls gesammelt, und dazu verwendet, ein L-Valin enthaltendes Produkt in flüssiger oder fester Form bereitzustellen. Hierfür wird auch der Ausdruck "Gewinnen des Feinchemikalie haltigen Produktes" verwendet. Im einfachsten Fall stellt die dem Fermentationsbehälter entnommene Feinchemikalie-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung des L-valins. Auf diese Weise werden Produkte isoliert, die einen gewünschten Gehalt an der Verbindung aufweisen.

Die teilweise (> 0% bis < 80%) bis vollständige (100%) oder nahezu vollständige (≥ 80% bis < 100%) Entfernung des Wassers (Maßnahmen a)) wird auch als Trocknung bezeichnet.

In einer Variante des Verfahrens gelangt man durch vollständige oder nahezu vollständige Entfernung des Wassers, der Biomasse, der organischen Nebenprodukte und der nicht verbrauchten Bestandteile des eingesetzten Fermentationsmediums zu reinen ((≥ 80 Gew.-%, ≥ 90 Gew.-%) oder hochreinen (≥ 95 Gew.-%, ≥ 97 Gew.-%, ≥ 99% Gew.-%) Produktformen des L-Valins. Für die Maßnahmen gemäß a), b), c) oder d) sind im Stand der Technik eine Fülle von technischen Anleitungen verfügbar.

Bei Verfahren zur Herstellung von L-Valin unter Verwendung von Bakterien der Gattung Corynebacterium werden solche Verfahren bevorzugt, bei denen Produkte erhalten werden, die keine Bestandteile der Fermentationsbrühe enthalten. Diese werden insbesondere in der Humanmedizin, in der pharmazeutischen Industrie und in der Lebensmittelindustrie verwendet.

Das erfindungsgemäße Verfahren dient zur fermentativen Herstellung von L-Valin.

Gegenstand der Erfindung ist schließlich die Verwendung des erfindungsgemäßen Mikroorganismus zur fermentativen Herstellung von L-Valin.

Die vorliegende Erfindung wird im Folgenden anhand von Ausführungsbeispielen näher erläutert.

### Beispiel 1 : (nicht erfindungsgemäß)

### Klonierung des Austauschkonstruktes pK18mobsacB PprpD2-ilvB

Ausgehend von der Genomsequenz von Corynebacterium glutamicum ATCC14067 wurde ein 1390 bp großes DNA-Fragment bei der Firma Life Technologies GmbH (Darmstadt,

Deutschland) synthetisiert (Seq ID NO: 5), welches aus den folgenden Komponenten besteht:
- homologer DNA-Bereich stromaufwärts von ilvB
- PprpD2-Promotor aus C. glutamicum ATCC14067
- Ribosomenbindestelle des gap-Gens aus C. glutamicum ATCC14067
- homologer Bereich zum ilvB-Gen, der anstelle des GTG-Startcödons ein ATG-Startcodon trägt.

Das Fragment wurde über die terminal eingeführten Schnittstellen EcoRI und HindIII durch jeweilige Spaltung mit den beiden genannten Restriktionsenzymen und anschließender Ligation in den analog mit EcoRI und HindIII gespaltenen Vektor pK18mobsacB kloniert. Das Plasmid trägt die Bezeichnung pK18mobsacB_PprpD2-ilvB. Es erlaubt die Herstellung einer Mutante in dem der native Promotor des ilvB-Gens deletiert und durch den indüzierbaren Promotor PprpD2 ersetzt wird. Dabei wird außerdem das native Start-Codon (GTG) durch das vom Ribosom bevorzugten Start-Codon ATG ersetzt.

### Beispiel 2 (nich erfindungsgemäß)

### Konstruktion des Austauschkonstruktes pK18mobsacB ilvN(M13)

Ausgehend von der Genomsequenz von Corynebacterium glutamicum ATCC14067 wurde ein 1421 bp großes DNA-Fragment bei der Firma Life Technologies GmbH (Darmstadt, Deutschland) synthetisiert (Seq ID NO: 14), welches einen Teil des ilvB-Gens, den intergenen Bereich zwischen ilvB-Gen und ilvN-Gen, sowie einen Teil des ilvN-Gens umfasst. Dabei wurde die native Sequenz "GGAATCATT" im ilvN-Gen (+58 bis +66 bp stromabwärts des Genstarts von ilvN, wobei der Genstart als +1 definiert ist) verändert zu "GATGACTTT". Dadurch wird die Aminosäuresequenz des IlvN-Proteins an den Positionen 20,21 und 22 verändert von Gly (20), Ile(21), Ile(22) zu Asp(20), Asp(21), Phe(22).

Das Fragment wurde über die terminal eingeführten Schnittstellen EcoRI und HindIII durch jeweilige Spaltung mit den beiden genannten Restriktionsenzymen und anschließender Ligation in den analog mit EcoRI und HindIII gespaltenen Vektor pK18mobsacB ligiert. Das Plasmid trägt die Bezeichnung pK18mobsacB_ilvN(M13). Es erlaubt die Herstellung einer Mutante, in der die native Gensequenz "GGAATCATT" (+58 bis +66 bp stromabwärts des Genstarts von ilvN, wobei der Genstart als +1 definiert ist) zu "GATGACTTT" verändert wird.

### Beispiel 3 : (nicht erfindungsgemäß)

### Konstruktion der Mutanten C. glutamicum ATCC14067 PprpD2-ilvBN, C. glutamicum ATCC14067 ilvN(M13) PprpD2-ilvBN und C. glutamicum VPS PprpD2-ilvBN.

Der in Beispiel 1 genannte Vektor pK18mobsacB_PprpD2-ilvB wurde mittels Elektroporation nach einem Protokoll von Liebl et al. (FEMS Microbiology Letters 65, 299-304 (1989) in den Stamm Corynebacterium glutamicum ATCC14067 und in die Valinproduktionsstämme Corynebacterium glutamicum ATCC14067_ilvN(M13) (siehe Beispiel 4) und Corynebacterium glutamicum-Valinproduktionsstamm C. glutamicum VPS transferiert. Der Vektor pK18mobsacB bzw. pK18mobsacB_PprpD2-ilvB kann in C. glutamicum ATCC14067, C. glutamicum ATCC14067_ilvN(M13) und C. glutamicum VPS nicht selbständig replizieren und bleibt nur dann in der Zelle erhalten, wenn er als Folge eines Rekombinationsereignisses ins Chromosom integriert hat. Die Selektion von Klonen mit integriertem pK18mobsacB_PprpD2_ilvB erfolgt durch Ausplattieren des Konjugationsansatzes auf LB-Agar (Sambrook et al., Molecular Cloning: A Laboratory Manual, 2nd Ed., Cold Spring Habor, New York, 1989), der mit 15 mg/l Kanamycin und 50 mg/ml Nalidixinsäure supplementiert worden ist. Angewachsene Klone werden auf LB-Agarplatten mit 25 mg/l Kanamycin ausgestrichen und für 16 Stunden bei 33°C inkubiert. Zur Selektion von Mutanten, bei denen als Folge eines zweiten Rekombinationsereignisses die Exzision des Plasmides stattgefunden hat, werden die Klone 20 Stunden unselektiv in LB-Flüssigmedium kultiviert, anschließend auf LB-Agar mit 10% Sucrose ausgestrichen und 24 Stunden bebrütet.

Das Plasmid pK18mobsacB_PprpD2-ilvB enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression führt zur Bildung der Levan-Sucrase, die die Synthese des für C. glutamicum toxischen Produktes Levan katalysiert. Auf LB-Agar mit Sucrose wachsen daher nur solche Klone an, bei denen das integriert pK18mobsacB_PprpD2-ilvB wiederum exzisiert hat. Bei der Exzision kann zusammen mit dem Plasmid entweder die vollständige wildtypische Kopie des ilvB-Gens inklusive des wildtypischen Promotorbereiches exzisieren, oder die rekombinante Kopie des ilvB-Gens mit dem PprpD2-Promotor.

Ungefähr 40 bis 50 Kolonien wurden auf den Phänotyp "Wachstum in Gegenwart von Sucrose" und "Nicht-Wachstum in Gegenwart von Kanamycin" geprüft. Um nachzuweisen, dass das rekombinante PprpD2-ilvB-Allel im Chromosom verblieben ist, wurden ungefähr 20 Kolonien, die den Phänotyp "Wachstum in Gegenwart von Sucrose" und "Nicht-Wachstum in Gegenwart von Kanamycin" aufweisen, nach der Standard-PCR-Methode von Innis et al. (PCR Protocols. A Guide to Methods and Applications, 1990, Academic Press) mit Hilfe der Polymerase-Kettenreaktion untersucht. Hierbei wurde aus der chromosomalen DNA der Kolonien ein DNA-Fragment amplifiziert, welches die modifizierten Bereiche des rekombinanten PprpD2-ilvB-Allels trägt. Es wurden die folgenden Primer-Oligonukleotide für die Nachweis-PCR ausgewählt.
Testprimer 1 (SEQ ID NO: 6)
   5'-AAA GCC TGC ATC GCG GAG AC-3'
Testprimer 2 (SEQ ID NO: 7)
   5'-TGG TGA TGC CGC GGA TAT CG-3'

Die Primer ermöglichen bei Klonen mit rekombinantem PprpD2-ilvBN -Lokus die Amplifizierung eines ca. 880 bp großen DNA-Frägmentes. Bei Klonen mit wildtypischem PilvBN-ilvBN Lokus werden DNA-Fragmente mit einer Größe von ca. 1136 bp amplifiziert.

Die amplifizierten DNA-Fragmente werden mittels Elektrophorese in einem 0,8%igen Agarosegel identifiziert. Damit konnte gezeigt werden, daß die Stämme ein modifiziertes, rekombinantes PprpD2-ilvBN-Allel auf dem Chromosom tragen. Die Stämme wurden als Corynebacterium glutamicum ATCC14067_PprpD2-ilvBN, ATCC14067_ilvN(M13)_PprpD2-ilvBN und VPS_PprpD2-ilvBN bezeichnet.

### Beispiel 4 (nicht erfindungsgemäß)

### Konstruktion des Stammes C. glutamicum ATCC14067 ilvN(M13)

Der in Beispiel 2 genannte Vektor pK18mobsacB_ilvN(M13) wurde analog zu der in Beispiel 3 beschriebenen Methode durch Elektroporation in den Stamm Corynebacterium glutamicum ATCC14067 transferiert. Die Selektion der Klone erfolgte durch die in Beispiel 3 genannten Kultivierungstechniken. Der Nachweis positiver Klone erfolgte auf Basis von chromosomaler DNA, die aus 20 Klonen isoliert worden war, durch Amplifikation eines 947 bp langen PCR-Produktes durch Polymerasekettenreaktion mit den Testprimern 3 und 4
Testprimer 3 (SEQ ID NO: 15)
   5'- CCC AGT AGT CAT CGA CTT C -3'
Testprimer 4 (SEQ ID NO: 16)
   5'- CAG CGT CAG CAT CAT AAA GC -3'
und anschließender Sequenzierung des PCR-Produktes.

### Beispiel 5:

### Leistungstest mit Corynebacterium glutamicum ATCC14067 PprpD2-ilvBN zur Herstellung von L-valin

Zur Untersuchung ihrer Fähigkeit L-Valin zu produzieren wurden fünf Klone des Stammes Corynebacterium glutamicum ATCC14067_PprpD2-ilvBN und als Referenz der Stamm Corynebacterium glutamicum ATCC14067 in jeweils 10 ml Testmedium für 16 h bei 33°C vorkultiviert. Für den Produktionstest wurden mit der erhaltenen Vorkultur je 10 ml Testmedium so angeimpft, dass die Start-OD₆₀₀ (optische Dichte bei 600 nm) 0,1 betrüg.. Jeder Klon wurde in drei Schüttelkolben geprüft, sodass der Beispielstamm durch insgesamt fünfzehn Schüttelkolben repräsentiert ist.

Das Testmedium war identisch mit dem bei Keilhauer et al. (Journal of Bacteriology (1993) 175: 5593-5603) beschriebenen CgXII-Medium, enthielt aber zusätzlich 7,5 g/l Hefeextract (Difco (Becton Dickinson GmbH), Heidelberg). Die Zusammensetzung des Testmediums ist in der nachfolgenden Tabelle 1 zusammengefasst. Das Testmedium zur Induktion der Valinsynthese enthielt zusätzlich Propionat in einer Konzentration von 0,6 g/l (bezogen auf die freie Säure).

**Tabelle 1**

| Komponente | Gehalt pro l |
|---|---|
| (NH₄)₂SO₄ | 20 g |
| Harnstoff | 5 g |
| KH₂PO₄ | 1 g |
| K₂HPO₄ | 1 g |
| MgSO₄ x 7 H₂O | 0,25 g |
| 3-Morpholinopropansulfonsäure (MOPS) | 42 g |
| CaCl₂ | 0,01 g |
| FeSO₄ x 7H₂O | 0,01 g |
| MnSO₄ x H₂O | 0,01 g |
| ZnSO₄ x 7 H₂O | 0,001 g |
| CuSO₄ | 0,0002 g |
| NiCl₂ x 6H₂O | 0,00002 g |
| Biotin | 0,0002 g |
| Protokatechusäure | 0,03 g |
| Dextrose | 40 g |
| Hefeextrakt | 7.5 g/l |
| pH (mit NaOH) | 7 |

Die Kultivierung erfolgte bei 33°C und 200 rpm in 100 ml Schüttelkolben. Die Auslenkung des Schüttlers war 5 cm. Nach 24 und 48 Stunden wurden Proben aus den Kulturen entnommen und die optische Dichte der Gehalt an Dextrose und der Gehalt an L-Valin bestimmt und die Zellen kurz abzentrifugiert (Tischzentrifuge Typ 5415D (Eppendorf) bei 13000 rpm, 10 min, Raumtemperatur).

Die Bestimmung der optischen Dichte erfolgte bei einer Wellenlänge von 660 nm mit einem GENios Mikrotiterplatten Photometer (Tecan, Reading UK). Die Proben wurden vor der Messung 1:100 mit demineralisiertem Wasser verdünnt.

Die Bestimmung der Dextrose erfolgte mit einem gekoppeltem Enzymtest (Hexokinase/Glukose-6-Phosphat Dehydrogenase) via NADH-Bildung.

Die quantitative Bestimmung der extrazellulären Aminosäurekonzentrationen aus dem Kulturüberstand erfolgte mittels reversed phase HPLC (Lindroth et al., Analytical chemistry (1979) 51: 1167-1174). Es wurde ein HPLC-Gerät der Serie HP1100 (Hewlett-Packard, Waldbronn, Deutschland) mit angeschlossenem Fluoreszenzdetektor (G1321A) verwendet; die Systemsteuerung und die Auswertung der Daten erfolgte mit einer HP-Chem-Station (Hewlett-Packard). 1 µL der zu analysierenden Aminosäurelösung würde in einer automatischen Vorsäulenderivatisierung mit 20 µL ortho-Phthalaldehyd/2-Mercaptoethanol-Fertigreagenz (Pierce Europe BV, Oud-Beijerland, Niederlande) gemischt. Die dabei entstehenden fluoreszierenden, thiosubstituierten Isoindole (Jones et al., Journal of Chromatography (1983) 266: 471-482) wurden über eine kombinierte Vorsäule (40x4 mm Hypersil ODS 5) und Hauptsäule (Hypersil ODS 5, beide Säulen von der Firma CS-Chromatographie Service GmbH, Langerwehe, Deutschland) mit einem Gradientenprogramm mit zunehmend unpolarer Phase (Methanol) aufgetrennt. Das polare Eluenz war Natriumacetat (0,1 M; pH 7,2); die Flußrate betrug 0,8 mL pro Minute. Die Fluoreszenzdetektion der derivatisierten Aminosäuren erfolgte bei einer Anregungswellenlänge von 230 nm und einer Emissionswellenlänge von 450 nm. Die Valin-Konzentrationen wurden durch einen Vergleich mit einem externen Standard berechnet.

Zur Berechnung der Ausbeute wurde die Menge des gebildeten L-Valins durch die Menge an verbrauchter Dextrose geteilt.

Die Ergebnisse sind in Tabelle 2 dargestellt und zeigen, dass der Beispielstamm Corynebacterium glutamicum ATCC14067_PprpD2-ilvBN in Gegenwart von Propionat im Medium signifikant Valin ausscheidet, während er sich ohne Propionat nicht vom Kontrollstamm Corynebacterium glutamicum ATCC14067 unterscheidet, der unter keiner Bedingung signifikant Valin produziert.

Tabelle 2: L-Valin Bildung nach 24 Stunden Inkubation ohne Propionsäure im Medium (Tabelle 2A) bzw, mit 0,6 g/l Propionsäure im Medium (Tabelle 2B). Abkürzungen: *: ATCC 14067_PprpD2-ilvBN, Stabw: Standardabweichung.

**Tabelle 2A: Ergebnisse ohne Propionsäure**

| Zeit | 24 Stunden | | |
|---|---|---|---|
| Stamm | Valin g/l (± Stabw) | Ausbeute g/g (± Stabw) | OD (± Stabw) |
| *_1 | < 0,2 | 0 | 26,2 ± 0,8 |
| *_2 | < 0,2 | 0 | 26,2 ± 1,7 |
| *_3 | < 0,2 | 0 | 26, 9 ± 2,2 |
| *_4 | < 0,2 | 0 | 27,0 ± 0,5 |
| *_5 | < 0,2 | 0 | 26,3 ± 1,8 |
| ATCC 14067 | < 0,2 | 0 | 26, 6 ± 0,4 |

**Tabelle 2B: Ergebnisse mit Propionsäure**

| Zeit | 24 Stunden | | |
|---|---|---|---|
| Stamm | Valin g/l (± Stabw) | Ausbeute g/g (± Stabw) | OD (± Stabw) |
| *_1 | 3,04 ± 0,13 | 0,07 ± 0,004 | 24,6 ± 0,3 |
| *_2 | 3,06 ± 0,10 | 0,07 ± 0,003 | 27,5 ± 0,8 |
| *_3 | 2,78 ± 0,06 | 0,07 ± 0,001 | 26,2 ± 0,4 |
| *_4 | 2,93 ± 0,03 | 0,07 ± 0,001 | 27,1 ± 1,4 |
| *_5 | 2,83 ± 0,04 | 0,07 ± 0,001 | 28,1 ± 0,1 |
| ATCC 14067 | < 0,2 | 0 | 26,2 ± 4,5 |

### Beispiel 6:

### Leistungstest mit den Corynebacterium glutamicum-Valin-Produktionsstämmen

Analog zu Beispiel 5 wurden die Stämme Corynebacterium glutamicum ATCC14067_ilvN(M13)_PprpD2-ilvBN und Corynebacterium glutamicum VPS_PprpD2-ilvBN im Schüttelkolbensystem untersucht.

Zur Untersuchung ihrer Fähigkeit L-Valin zu produzierten wurden der Stamm Corynebacterium glutamicum ATCC14067_ilvN(M13)_PprpD2-ilvBN und als Referenz der Stamm Corynebacterium glutamicum ATCC14067_ilvN(M13) bzw. der Stamm Corynebacterium glutamicum VPS_PprpD2-ilvBN und als Referenz der Stamm Corynebacterium glutamicum VPS in jeweils 10 ml Testmedium (Tabelle 3) für 16 h bei 33°C vorkultiviert. Für den Produktionstest wurden mit der erhaltenen Vorkultur je 10 ml Testmedium so angeimpft, dass die Start-OD₆₀₀ (optische Dichte bei 600 nm) 0,1 betrug. Jeder Klon wurde in drei Schüttelkolben geprüft, sodass der Beispielstamm durch insgesamt fünfzehn Schüttelkolben repräsentiert ist.

**Tabelle 3: Als Testmedium wurde SK1039 verwendet**

| | Konzentration (g/l) |
|---|---|
| Glucose | 40,0 |
| (NH₄)₂SO₄ (100% TDM) | 8,5 |
| MgSO₄*7H₂O | 0,85 |
| KH₂PO₄ | 0,2 |
| Hefeextrakt | 1,14 |
| CSL | 10,0 |
| MOPS | 20,0 |
| D-(+)-Biotin 2% | 0,00425 |
| Thiamin HCl | 0,00119 |
| *Fe*-*Sulfat 7H2O* | 0,003 |
| *MnSO4 H2O* | 0,003 |
| CaCo3 | 10,00 |

Das Testmedium zur Induktion der Valinsynthese enthielt zusätzlich Propionat in einer Konzentration von 0,6 g/l (bezogen auf die freie Säure).

Die Kultivierungsbedingungen, die Bestimmung der Biomasse, der Dextrose und der Valinkonzentration erfolgten analog wie in Beispiel 5 beschrieben.

Die Ergebnisse sind in Tabelle 4 dargestellt und zeigen, dass die Valin-Produkfipnsstämme Corynebacterium glutamicum ATCC14067_ilvN(M13)_PprpD2-ilvBN und VPS_PprpD2-ilvBN in Gegenwart von Propionat im Medium eine höhere spezifische Ausbeute an Valin in Bezug auf die eingesetzte Kohlenstoffquelle besitzen als die jeweils nicht modifizierten Ausgangsstämme Corynebacterium glutamicum ATCC14067_ilvN(M13) bzw. Corynebacterium glutamicum VPS.

Tabelle 4: L-Valin-Ausbeute nach 24 Stunden Inkubation ohne Propionsäure im Medium (Tabelle 4A) bzw. mit 0,6 g/l Propionsäure im Medium (Tabelle 4B); Abkürzungen: Stabw = Standardabweichung.

**Tabelle 4A: Ergebnisse ohne Propionsäure**

| Stamm | Ausbeute g/g (± Stabw) |
|---|---|
| ATCC14067_ilvN(M13)_PprpD2-ilvBN | 0,02 ± 0,00 |
| ATCC14067_ilvN(M13) | 0,35 ± 0,01 |
| VPS_PprpD2-ilvBN | 0,02 ± 0,00 |
| VPS | 0,40 ± 0,02 |

**Tabelle 4B: Ergebnisse mit Propionsäure**

| Stamm | Ausbeute g/g (± Stabw) |
|---|---|
| ATCC14067_ilvN(M13)_PprpD2-ilvBN | 0,42 ± 0,02 |
| ATCC14067_ilvN(M13) | 0,36 ± 0,01 |
| VPS_PprpD2-ilvBN | 0,45 ± 0,02 |
| VPS | 0,41 ± 0,01 |

### Beispiel 7:

### Valin-Stabilitätstest für die Stämme VPS PprpD2-ilvBN und VPS

Der Stabilitätstest wurde in 10 ml Flüssigkulturen (Wie in Beispiel 6) durchgeführt.

Vorkultivierung der Stämme VPS und VPS_PprpD2-ilvBN 10 ml Flüssigkultur in einem 100 ml Schüttelkolben (mit Schikanen) wurden mit je 50 µl aus einer Glycerin-Dauerkultur angeimpft und 22 h inkubiert (33°C, 200 rpm, 5cm Amplitude).

Die optische Dichte der Kulturen wurde gemessen und 1.5 ml der Kultur wurden mit Glycerin versetzt (10% Glycerin Endkonzentration) und als Kryokultur bei -80°C in einem Schraubdeckelgefäß eingefroren.

Mit 50 µl der Kulturen wurde je eine neue 10 ml Flüssigkultur beimpft und diese erneut für 22 h bei 33°C unter Schütteln inkubiert.

Diese Prozedur wurde noch zweimal wiederholt, so dass jede Glycerinkultur ingesamt in vier aufeinanderfolgenden Flüssigkülturen kultiviert wurde. Jede Kultivierung entspricht ca. 8 Zellgenerationen. Die vier Passage in Flüssigkulturen entsprechen also insgesamt über 30 (ca. 32) Zellgenerationen.

### Hauptkultivierung der Stämme VPS und VPS_PprpD2-ilvBN

Von jeder Dauerkultur bzw. Kryokultur wurden Schüttelkolbenkulturen mit je 10 ml Flüssigmedium auf eine Start-OD von 0.1 angeimpft. Die Kulturen wurden im Anschluss 24 h inkubiert. Jede Kultivierung wurde in einer Doppelbestimmung durchgeführt. Am Ende der Inkubation (nach 24 h) wurden Proben zur Analyse der optischen Dichte, des Valintiters und der Restzuckerkonzentration genommen. Die Analysen wurden wie unter Beispiel 5 beschrieben durchgeführt.

Im Ergebnis des Leistungstests (Tab. 5) wird gezeigt, dass mit jeder Passage der Valintiter (angegeben in relativer Veränderung in Prozent), das Valin/Biomasse-Verhältnis (angegeben in Valin/OD) und die Ausbeute (g gebildetes Produkt/ g verbrauchtes Substrat) für den Stamm VPS schlechter bzw. niedriger werden. Dies ist ein Beleg dafür, dass sich in der Population Mutationen etablieren, die negativ für die Produktbildung und positiv für die Biomassebildung sind. Der Stamm VPS zeigt bereits nach zwei Anzuchtstufen (15,6 Generationen) ein Abfallen der Leistungsdaten in der Testkultivierung (Ergebnisse Hauptkultivierung). Damit wäre in einem 4-stufigen Verfahren (3 Anzuchtstufen + 1 Produktionsstufe) ein starker Rückgang der Leistungsdaten (Titer, Ausbeute, Biomassespezifische Produktbildung) zu erwarten. Für den erfindungsgemässen Stamm VPS_PprpD2-ilvBN zeigt sich hingegen auch bei Durchlaufen von vier Anzuchtstufen mit mehr als 30 Generationen dieser negative Effekt nicht. Im Gegenteil dazu steigt die Biomassenspezifische Valinbildung (Valin/OD) sogar leicht an. Damit wird mindestens ein 4-stufiges Produktionsverfahren bestehend aus 3 Anzuchtstufen und einer Hauptkultivierung simuliert bzw. die Anforderung sogar übertroffen.

**Tabelle 5: Leistungsdaten des Stabilitätstests (L-Valin-Ausbeute, biomasse-spezifische Produktbildung (Valin/OD) und relative Veränderungen der Valinbildung in Abhängigkeit der zusätzlichen Zellgenerationen zur Kontrolle (=0) nach 24 Stunden Inkubation (Mittelwerte)**

| | | Ergebnisse der Hauptkultivierung | | |
|---|---|---|---|---|
| Stamm | Zusätzliche Zell-Generationen in der Anzuchtschiene im Vergleich zur Kontrolle | relative Änderung der Valin-Bildung im Vergleich zur Kontrolle | Valin/Optische Dichte (OD) | Ausbeute Valin/Dextrose Y (P/S) [g/g] |
| | | [%] | | [%] |
| VPS_PprpD2-ilvBN (= kontrolle) | 0 | 0 | 1,8 | 54% |
| VRS_PprpD2-ilvBN_1 | 9 | -3 | 2,1 | 64% |
| VPS_PprpD2-ilvBN_2 | 17 | 6 | 2,8 | 60% |
| VPS_PprpD2-ilvBN_3 | 24 | -5 | 2,3 | 60% |
| VPS_PprpD2-ilvBN_4 | 32 | 3 | 2,4 | 63% |
| VPS (= Kontrolle) | 0 | 0 | 2,7 | 45% |
| VPS_1 | 7,7 | 2 | 2,9 | 42% |
| VPS_2 | 15,6 | -58 | 2,5 | 48% |
| VPS_3 | 23,4 | -78 | 1,5 | 44% |
| VPS_4 | 31,7 | -85 | 1,0 | 30% |

- Figur 1:: Karte des Plasmids pK18mobsacB_PprpD2-ilvB

Die verwendeten Abkürzungen und Bezeichnungen haben folgende Bedeutung.
- oriV:: ColE1-ähnlicher Origin aus pMB1
- sacB:: das für das Protein Levansucrose kodierende sacB-Gen
- RP4mob:: RP4-Mobilisierungs-Site
- Kan:: Resistenzgen für Kanamycin
- PprpD2:: Propionat induzierbarer Promotor
- 'ilvB:: 5'-Region des ilvB Gens
- HindIII:: Schnittstelle des Restriktionsenzyms HindIII
- EcoRI:: Schnittstelle des Restriktionsenzyms EcoRI

## Patentansprüche

1. Verfahren zur Herstellung von L-Valin durch Fermentation von Mikroorganismen der Gattung Corynebacterium, enthaltend in replizierbarer Form ein Polynukleotid mit Operatoraktivität, dessen Sequenz zu mindestens 85% identisch ist mit der Sequenz von Position 1 bis 121 gemäß SEQ ID NO: 1, 2 oder 3, an welches der Aktivator PrpR bindet, und dem am 3'-Ende ein zweites Polynukleotid mit Promotoraktivität sowie die für die Untereinheiten einer Acetolactat-Synthase kodierenden Gene ilvB und ilvN funktionsfähig nachgeschaltet sind und das die Transkription der Gene ilvBN in Abhängigkeit von der Anlagerung des Aktivators PrpR reguliert,
in einem Medium, dem nach einer ersten Phase, der Wachstumsphase, welche ohne Induktor stattfindet, in einer zweiten Phase als Induktor Propionat oder 2-Methylcitrat zugegeben wird, worauf L-Valin synthetisiert wird, unter Bedingungen, bei denen L-Valin im Medium und/oder in den Zellen angereichert wird.

2. Verfahren nach Anspruch 1, **dadurch gekennzeichnet, dass** das Polynukleotid mit Operatoraktivität ein Polynukleotid ("IR 1") umfasst, dessen Sequenz zu mindestens 90 % identisch ist zu der Sequenz gemäß Position 22 bis Position 49 gemäß SEQ ID NO: 1, 2 oder 3, sowie ein Polynukleotid ("IR 2"), dessen Sequenz zu mindestens 90 % identisch ist zu der Sequenz von Position 77 bis Position 105 gemäß SEQ ID NO: 1, 2 oder 3.

3. Verfahren nach Anspruch 1 oder 2, **dadurch gekennzeichnet, dass** es sich bei dem Polynukleotid mit Operatoraktivität um ein Polynukleotid mit einer Sequenz handelt, die zu mindestens 90 % identisch ist zu der Sequenz von Position 1 bis 121 gemäß SEQ ID NO: 1, 2 oder 3.

4. Verfahren nach einem der vorhergehenden Ansprüche, **dadurch gekennzeichnet, dass** es sich bei dem Polynukleotid mit Promotoraktivität um ein Polynukleotid mit einer Sequenz handelt, die zu mindestens 90 % identisch ist zu der Sequenz von Position 122 bis 206 gemäß SEQ ID NO: 4.

5. Verfahren nach einem der vorhergehenden Ansprüche, **dadurch gekennzeichnet, dass** es sich bei dem Gen ilvB um ein Polynukleotid handelt, das für ein Polypeptid kodiert, dessen Sequenz zu mindestens 90 % identisch ist zu der Sequenz gemäß SEQ ID NO: 9 und dass es sich bei dem Gen ilvN um ein Polynukleotid handelt, das für ein Polypeptid kodiert, dessen Sequenz zu mindestens 90 % identisch ist zu der Sequenz gemäß SEQ ID NO: 10.

6. Verfahren nach einem der vorhergehenden Ansprüche, **dadurch gekennzeichnet, dass** man Mikroorganismen einsetzt, in denen zusätzlich weitere Enzyme des Biosyntheseweges von L-Valin verstärkt vorliegen und/oder in denen die Stoffwechselwege zumindest teilweise abgeschwächt sind, die die Bildung von L-Valin verringern.

7. Verfahren nach einem der vorhergehenden Ansprüche, **dadurch gekennzeichnet, dass** die eingesetzten Corynebakterien in der Anzuchtphase mindestens 16 Generationen durchlaufen.

8. Verfahren nach einem der vorhergehenden Ansprüche, **dadurch gekennzeichnet, dass** die Fermentation mindestens vier Stufen, bestehend aus Schüttelkolben, PreSeed-Fermenter, Seed-Fermenter und Produktions-Fermenter, umfasst.

9. Mikroorganismus, der L-Valin produziert, enthaltend in replizierbarer Form eine Expressionskassette und/oder einen diese Expressionskassette enthaltenden Vektor, wobei die Expressionskassette folgende Elemente umfasst: ein Polynukleotid mit Operatoraktivität, dessen Sequenz zu mindestens 85 % identisch ist zu der Sequenz von Position 1 bis 121 gemäß SEQ ID NO: 1, 2 oder 3, einen nachgeschalteten Promotor sowie die für eine Acetolactat-Synthase kodierenden Gene ilvBN.

10. Mikroorganismus nach Anspruch 9, **dadurch gekennzeichnet, dass** der nachgeschaltete Promotor eine Sequenz aufweist, die zu mindestens 90 % identisch ist zu der Sequenz von Position 122 bis 206 gemäß SEQ ID NO: 4.

11. Mikroorganismus nach Anspruch 9 oder 10, **dadurch gekennzeichnet, dass** es sich um einen Mikroorganismus der Gattung Corynebacterium handelt.

12. Mikroorganismus nach Anspruch 11, **dadurch gekennzeichnet, dass** es sich um einen Mikroorganismus der Spezies Corynebacterium glutamicum handelt.

13. Mikroorganismus nach einem der vorhergehenden Ansprüche, dadurch gekenntzeichnet, dass die Expressionskassette in das Chromosom des Mikroorganismus integriert wurde.

14. Mikroorganismus nach einem der vorhergehenden Ansprüche, **dadurch gekennzeichnet, dass** zusätzlich weitere Enzyme des Biosyntheseweges von L-Valin verstärkt vorliegen und/oder die Stoffwechselwege zumindest teilweise abgeschwächt sind, die die Bildung des L-Valins verringern.

## Claims

1. Process for the preparation of L-valine by fermentation of microorganisms of the genus Corynebacterium, comprising, in replicable form, a polynucleotide with operator activity whose sequence is to at least 85% identical to the sequence of position 1 to 121 according to SEQ ID NO: 1, 2 or 3 to which the activator PrpR binds and which has operably arranged downstream at the 3' end a second polynucleotide with promoter activity and the genes ilvB and ilvN encoding the subunits of an acetolactate-synthase and which regulates the transcription of the genes ilvBN as a function of the attachment of the activator PrpR,
in a medium to which, after a first phase, the growth phase, which takes place without inductor, is added, in a second phase, propionate or 2-methyl citrate as inductor, whereupon L-valine is synthesised, under conditions at which L-valine is accumulated in the medium and/or in the cells.

2. Process according to Claim 1, **characterized in that** the polynucleotide with operator activity comprises a polynucleotide ("IR 1") whose sequence is to at least 90% identical to the sequence according to position 22 to position 49 according to SEQ ID NO: 1, 2 or 3, and a polynucleotide ("IR 2") whose sequence is to at least 90% identical to the sequence of position 77 to position 105 according to SEQ ID NO: 1, 2 or 3.

3. Process according to Claim 1 or 2, **characterized in that** the polynucleotide with operator activity is a polynucleotide with a sequence which is to at least 90% identical to the sequence of position 1 to 121 according to SEQ ID NO: 1, 2 or 3.

4. Process according to one of the preceding claims, **characterized in that** the polynucleotide with promoter activity is a polynucleotide with a sequence which is to at least 90% identical to the sequence of position 122 to 206 according to SEQ ID NO: 4.

5. Process according to one of the preceding claims, **characterized in that** the gene ilvB is a polynucleotide which codes for a polypeptide whose sequence is to at least 90% identical to the sequence according to SEQ ID NO: 9 and that the gene ilvN is a polynucleotide which codes for a polypeptide whose sequence is to at least 90% identical to the sequence according to SEQ ID NO: 10.

6. Process according to one of the preceding claims, **characterized in that** microorganisms are employed in which additionally further enzymes of the L-valine biosynthetic pathway are present to a higher degree and/or in which the metabolic pathways reducing L-valine formation are attenuated at least in part.

7. Process according to one of the preceding claims, **characterized in that** the Corynebacteria employed go through at least 16 generations in the propagation phase.

8. Process according to one of the preceding claims, **characterized in that** the fermentation comprises at least four steps, consisting of shake flask, pre-seed fermenter, seed fermenter and production fermenter.

9. Microorganism which produces L-valine, comprising, in replicable form, an expression cassette and/or a vector comprising this expression cassette, the expression cassette comprising the following elements: a polynucleotide with operator activity whose sequence is to at least 85% identical to the sequence of position 1 to 121 according to SEQ ID NO: 1, 2 or 3, a promoter which is arranged downstream, and the ilvBN genes which code for an acetolactate synthase.

10. Microorganism according to Claim 9, **characterized in that** the promoter which is arranged downstream includes a sequence which is to at least 90% identical to the sequence of position 122 to 206 according to SEQ ID NO: 4.

11. Microorganism according to Claim 9 or 10, **characterized in that** it is a microorganism of the genus Corynebacterium.

12. Microorganism according to Claim 11, **characterized in that** it is a microorganism of the species Corynebacterium glutamicum.

13. Microorganism according to one of the preceding claims, **characterized in that** the expression cassette has been integrated into the chromosome of the microorganism.

14. Microorganism according to one of the preceding claims, **characterized in that** additionally further enzymes of the L-valine biosynthetic pathway are present to a higher degree and/or the metabolic pathways which reduce L-valine formation are attenuated, at least in part.

## Revendications

1. Procédé pour la production de L-valine par fermentation de micro-organismes du genre Corynebacterium, contenant sous forme réplicable un polynucléotide présentant une activité d'opérateur, dont la séquence est identique à raison d'au moins 85% à la séquence de la position 1 à la position 121 selon les séquences SEQ ID NO : 1, 2 ou 3, à laquelle se lie l'activateur PrpR, et qui est suivi, de manière fonctionnellement apte, en l'extrémité 3', d'un deuxième polynucléotide présentant une activité de promoteur ainsi que des gènes ilvB et ilvN codant pour les sous-unités d'une acétolactate synthase et qui régule la transcription des gènes ilvBN en fonction de l'addition de l'activateur PrpR, dans un milieu auquel est ajouté, après une première phase, la phase de croissance, qui a lieu sans inducteur, dans une deuxième phase, comme inducteur, du propionate ou du 2-méthylcitrate, suite à quoi de la L-valine est synthétisée, dans des conditions dans lesquelles la L-valine est enrichie dans le milieu et/ou dans les cellules.

2. Procédé selon la revendication 1, **caractérisé en ce que** le polynucléotide présentant une activité d'opérateur comprend un polynucléotide ("IR 1"), dont la séquence est identique à raison d'au moins 90% à la séquence de la position 22 à la position 49 selon les séquences SEQ ID NO : 1, 2 ou 3, ainsi qu'un polynucléotide ("IR 2"), dont la séquence est identique à raison d'au moins 90% à la séquence de la position 77 à la position 105 selon les séquences SEQ ID NO : 1, 2 ou 3.

3. Procédé selon la revendication 1 ou 2, **caractérisé en ce qu'**il s'agit, pour le polynucléotide présentant une activité d'opérateur, d'un polynucléotide présentant une séquence identique à raison d'au moins 90% à la séquence de la position 1 à la position 121 selon les séquences SEQ ID NO : 1, 2 ou 3.

4. Procédé selon l'une quelconque des revendications précédentes, **caractérisé en ce qu'**il s'agit, pour le polynucléotide présentant une activité de promoteur, d'un polynucléotide présentant une séquence identique à raison d'au moins 90% à la séquence de la position 122 à la position 206 selon la séquence SEQ ID NO : 4.

5. Procédé selon l'une quelconque des revendications précédentes, **caractérisé en ce qu'**il s'agit, pour le gène ilvB, d'un polynucléotide qui code pour un polypeptide dont la séquence est identique à raison d'au moins 90% à la séquence selon SEQ ID NO : 9 et **en ce qu'**il s'agit, pour le gène ilvN, d'un polynucléotide qui code pour un polypeptide dont la séquence est identique à raison d'au moins 90% à la séquence selon SEQ ID NO : 10.

6. Procédé selon l'une quelconque des revendications précédentes, **caractérisé en ce qu'**on utilise des micro-organismes dans lesquels se trouvent en outre d'autres enzymes de la voie de biosynthèse de la L-valine sous forme renforcée et/ou dans lesquels les voies métaboliques qui diminuent la formation de la L-valine sont au moins partiellement affaiblies.

7. Procédé selon l'une quelconque des revendications précédentes, **caractérisé en ce que** les corynebactéries utilisées traversent au moins 16 générations dans la phase de croissance.

8. Procédé selon l'une quelconque des revendications précédentes, **caractérisé en ce que** la fermentation comprend au moins quatre étapes, constituées par un ballon agité, un fermenteur de pré-ensemencement, un fermenteur d'ensemencement et un fermenteur de production.

9. Micro-organisme qui produit de la L-valine contenant, sous forme réplicable, une cassette expression et/ou un vecteur contenant cette cassette d'expression, la cassette d'expression comprenant les éléments suivants : un polynucléotide présentant une activité d'opérateur, dont la séquence est identique à raison d'au moins 85% à la séquence de la position 1 à la position 121 selon les séquences SEQ ID NO : 1, 2 ou 3, un promoteur disposé en aval ainsi que les gènes ilvBN codant pour une acétolactate synthase.

10. Micro-organisme selon la revendication 9, **caractérisé en ce que** le promoteur disposé en aval présente une séquence qui est identique à raison d'au moins 90% à la séquence de la position 122 à la position 206 selon la séquence SEQ ID NO : 4.

11. Micro-organisme selon la revendication 9 ou 10, **caractérisé en ce qu'**il s'agit d'un micro-organisme du genre Corynebacterium.

12. Micro-organisme selon la revendication 11, **caractérisé en ce qu'**il s'agit d'un micro-organisme de l'espèce Corynebacterium glutamicum.

13. Micro-organisme selon l'une quelconque des revendications précédentes, **caractérisé en ce que** la cassette d'expression a été intégrée dans le chromosome du micro-organisme.

14. Micro-organisme selon l'une quelconque des revendications précédentes, **caractérisé en ce qu'**en outre d'autres enzymes de la voie de biosynthèse de la L-valine se trouvent sous forme renforcée et/ou les voies métaboliques qui diminuent la formation de la L-valine sont au moins partiellement affaiblies.
